(19) Europäisches Patentamt
European Patent Office
Office européen des brevets

(11) **EP 4 045 138 B1**

(12) **EUROPEAN PATENT SPECIFICATION**

(45) Date of publication and mention
of the grant of the patent:
**25.03.2026 Bulletin 2026/13**

(21) Application number: **20876006.6**

(22) Date of filing: **18.10.2020**

(51) International Patent Classification (IPC):
*A61N 1/36* (2006.01)       *A61B 5/02* (2006.01)
*A61B 5/00* (2006.01)       *A61B 5/026* (2006.01)
*A61B 5/021* (2006.01)      *G06T 7/00* (2017.01)
*A61B 5/024* (2006.01)      *A61M 1/36* (2006.01)

(52) Cooperative Patent Classification (CPC):
**A61M 1/3656; A61B 5/0075; A61B 5/0077;**
**A61B 5/02007; A61B 5/02416; A61B 5/7264;**
**A61B 5/7275;** A61M 2205/3306

(86) International application number:
**PCT/IL2020/051100**

(87) International publication number:
**WO 2021/074920 (22.04.2021 Gazette 2021/16)**

(54) **SYSTEM FOR MONITORING THE FUNCTIONALITY OF A BLOOD VESSEL**

SYSTEM ZUR ÜBERWACHUNG DER FUNKTIONSFÄHIGKEIT EINES BLUTGEFÄSSES

SYSTÈME DE SURVEILLANCE DE LA FONCTIONNALITÉ D'UN VAISSEAU SANGUIN

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **18.10.2019 US 201916656585**

(43) Date of publication of application:
**24.08.2022 Bulletin 2022/34**

(73) Proprietor: **Patensee Ltd.**
**6037606 Or Yehuda (IL)**

(72) Inventors:
• **DRORI, Hagay**
**6219501 Tel-Aviv (IL)**
• **SEADIA, Oz Moshe**
**5964105 Bat Yam (IL)**
• **GOSHEN, Gal**
**7177955 Modiln (IL)**
• **LANGER, Ruben**
**4740025 Ramat HaSharon (IL)**

(74) Representative: **Regimbeau**
**20, rue de Chazelles**
**75847 Paris Cedex 17 (FR)**

(56) References cited:
**US-A1- 2014 148 658       US-A1- 2015 342 551**
**US-A1- 2017 119 258       US-A1- 2017 287 132**
**US-A1- 2018 000 354**

**Description**

FIELD AND BACKGROUND

The invention relates generally to the field of monitoring blood vessels in patients. Some aspects relate more particularly to early diagnosis of failure in blood vessel functionality, and even more particularly to early detection of failure of vascular access in patients undergoing hemodialysis treatments. Some aspects relate more particularly to measurements of fistulas.

Dialysis vascular access (VA, fistula or graft) makes life-saving hemodialysis treatments possible but also prone to access related problems.

The term "VA", or "vascular access", in all its grammatical forms, is used throughout the present specification and claims to mean all types of vascular access constructs, biological as well as synthetic, including, by way of some non-limiting examples, an arteriovenous fistula (AV), a synthetic graft, and an intravenous catheter.

One type of long-term access is an AV fistula. A surgeon connects an artery to a vein, usually in an arm or leg, to create an AV fistula. When the surgeon connects the artery to the vein, the vein grows wider and thicker, making it easier to place needles for dialysis. The AV fistula also has a large diameter that allows blood to flow out and back into a body quickly. A goal of an AV fistula is to allow high blood flow so that a large amount of blood can pass through a dialyzer.

- Over 25% of hospitalizations in hemodialysis (HD) patients in the United States are access related.
- Over 30,000 patients required dialysis-related arteriovenous thrombectomy in 2013.

The annual cost of access morbidity has been estimated at close to $1 billion.

VA function and patency are essential for optimal management of HD patients. Low VA flow and loss of patency limit hemodialysis delivery, extend treatment times, and may result in under-dialysis that leads to increased morbidity and mortality. In long-term VAs, especially grafts, thrombosis is the leading cause of loss of VA patency and increases healthcare expenditure.

The basic concept for VA monitoring and surveillance is that progressive stenoses develop over variable intervals in the great majority of VAs and, if detected and corrected (corrective procedure such as percutaneous transluminal angioplasty - PTA), under-dialysis can be minimized or avoided (dialysis dose protection) and the rate of thrombosis can be reduced. A number of monitoring and surveillance methods are available: sequential VA flow, sequential dynamic or static pressures, recirculation measurements, and physical examination.

Monitoring is the examination and evaluation of the VA to diagnose VA dysfunction using physical examination, usually within the HD unit, in order to detect the presence of dysfunction and correctable lesions before VA loss.

Physical examination can be used as a monitoring tool to exclude low flow associated with impending fistula and graft failures. Typically, there are 3 components to the VA examination: inspection, palpation, and auscultation.

A simple inspection can reveal the presence of swelling, ischemic fingers, aneurysms, and rich collateral veins. A strong pulse and weak thrill in the vein central to the anastomosis indicates a draining vein stenosis. Strictures can be palpated, and the intensity and character of the bruits can suggest the location of stenoses. A local intensification of bruit over the graft or the venous anastomosis compared with the adjacent segment suggests a stricture or stenosis. Physical examination can also include the elevation test, which consists of the elevation of the extremity with the VA and examination of the normal collapse of the access. The test is considered normal when the fistula collapses after the organ is elevated above the heart level of the patient.

Additional background art includes:

Besarab et al, "Access Monitoring Is Worthwhile and Valuable", Blood Purif 2006;24:77-89, February 2006;

Ehsan Rajabi-Jaghargh, Rupak K Banerjee, "Combined functional and anatomical diagnostic endpoints for assessing arteriovenous fistula dysfunction" World J Nephrol 2015 February 6; 4(1): 6-18 ISSN 2220-6124;

Luc Turmel-Rodrigues, "Salvage of immature forearm fistulas for haemodialysis by interventional radiology", Nephrol Dial Transplant. 2001 Dec;16(12):2365-71; and

Jürg Schmidli et al., Editor's Choice e Vascular Access: 2018 Clinical Practice Guidelines of the European Society for Vascular Surgery (ESVS) Eur J Vasc Endovasc Surg (2018) 55, 757e818.

Additional background art includes U.S. Patent Application Publication No. US2017/119258 disclosing systems and methods for assessing patient blood flow using video image processing, including capturing a video including a plurality of frames of an arterio-venous (AV) fistula on the patient; amplifying motion in the video to produce a motion-amplified video; determining a difference in intensity between consecutive frames in the motion-amplified video to produce a time-function of an amplitude of the optic flow representing movement in an area of interest on the patient; and determining the at least one blood flow characteristic of the patient based on the time-function.

U.S. Patent Application Publication No. US2014/148658 disclosing a system and method for use in monitoring one or more conditions of a subject's body. The system comprises a control unit which comprises an input port for receiving image data and data indicative of at least one external stimulation (external field) applied to a portion of the subject's body during collection of the image data therefrom, a memory utility, and a processor utility. The image data is indicative of a sequence of speckle patterns generated by the portion of the subject's body according to a certain sampling time pattern. The processor utility is configured and operable for carrying out the following: processing the image data utilizing the data indicative of the applied external field(s), said processing comprising determining a spatial correlation function between successive speckle patterns in the sequence, and determining a time varying spatial correlation function in the form of a time-varying function of at least one feature of the correlation function indicative of a change of the speckle pattern over time; selecting at least one parameter of the time-varying spatial correlation function, and applying to said at least one parameter one or more of the models to determine one or more corresponding body conditions; and generating output data indicative of said one or more corresponding body conditions.

U.S. Patent Application Publication No. US2018/000354 disclosing assessing cardiovascular function using an optical sensor, such as through sensing relevant hemodynamics understood by pulse transit times, blood pressures, pulse-wave velocities, and, in more breadth, ballistocardiograms and pressure-volume loops. The techniques disclosed in the document use various optical sensors to sense hemodynamics, such as skin color and skin and other organ displacement.

U.S. Patent Application Publication No. US2015/342551 disclosing a method for vascular assessment comprising receiving a plurality of 2-D angiographic images of a portion of a vasculature of a subject, and processing the images to produce a stenotic model over the vasculature, the stenotic model having measurements of the vasculature at one or more locations along vessels of the vasculature. The method further comprises obtaining a flow characteristic of the stenotic model, and calculating an index indicative of vascular function, based, at least in part, on the flow characteristic in the stenotic model.

U.S. Patent Application Publication No. US2017/287132 disclosing determining collateral information describing blood flow in collaterals of a blood vessel system in a target region of a patient from a four-dimensional vascular data set describing image values of temporal flow of a contrast medium and/or marked blood constituents as recorded by a medical imaging device. A method includes segmenting the blood vessel system in the vascular data set and determining collaterals among the segmented blood vessels by a collateral classifier. For all collaterals determined, a diameter of the collateral is determined taking into account the segmentation, a filling parameter describing the filling of the collaterals, and a time parameter describing the time response relative to a reference point in the blood vessel system from a temporal course of the image values in a portion of the collaterals under consideration. The method includes determining the collateral information from the diameter, the filling parameter, and the time parameter.

## SUMMARY OF THE INVENTION

The disclosure relates generally to automating monitoring of blood vessels in patients, more particularly to early diagnosis of failure in blood vessel functionality, and even more particularly to early detection of failure of vascular access in patients undergoing hemodialysis treatments.

According to the present invention there is provided a system (**200**) for monitoring vascular access in patients undergoing hemodialysis treatments by an automatic look listen feel examination, the system comprising:

> a camera (**204**) configured to image a patient's body (**212**) to obtain blood vessel geometry;
> an illumination source (**202**); and
> a processor (**208**) configured to receive an image from the camera (**204**),
> the system (**200**) configured to:

>> obtain a shape of a body organ at a location of a vascular access (VA) of the patient's body (**212**) using image analysis; and
>> analyze vibrations of the patient's body (**212**) at a location of the patient's body which includes blood vessels using image analysis,
>> characterized by
>> the system is programmed to automatically perform a look listen and feel examination of the patient's body by:

>>> implementing the look by image analysis of images obtained by the imaging;
>>> implementing the listen by the analyzing vibrations, at human audible frequencies, by image analysis of the images obtained by the imaging; and
>>> implementing the feel by the analyzing vibrations at frequencies extending to below human audible frequencies, by image analysis of the images obtained by the imaging.

According to some embodiments of the invention, the system comprises a classifier configured to combine two or more features extracted from the look, the listen and the feel, to classify a condition of the VA.

According to some embodiments of the invention, the obtain a shape of a body organ comprises calculating a three-dimensional (3D) shape of the patient's fistula.

According to some embodiments of the invention, the system is configured to record the features measured along a surveillance period and predicting likelihood of stenosis based on changes along the surveillance period.

According to some embodiments of the invention, the illumination source (202) is configured to switch projecting light between at least two out of three modes of projecting light: uniform illumination, structured light illumination and coherent laser light for spot illumination.

According to some embodiments of the invention, the system comprises a camera capable of capturing images at a frame rate greater than 150 Frames Per Second (FPS).

According to some embodiments of the invention, the illumination source (202) comprises a Digital Light Processing (DLP) projector.

According to some embodiments of the invention, the system is configured to calculate a rate of evacuation of a patient's fistula based on changes in a 3D shape of the patient's fistula.

According to some embodiments of the invention, said calculate is performed during an elevation test.

According to some embodiments of the invention, the system is configured to calculate one or more parameters indicative of development of one or more collateral vessels based upon the image analysis.

According to some embodiments of the invention, the system is configured to provide feedback to support a remote physical examination.

According to some embodiments of the invention, the system is configured to calculate one or more parameters based upon the image analysis and calculate an estimation of a maturity of a VA is calculated based on a rate of change of the one or more parameters.

According to some embodiments of the invention, the system is configured to calculate one or more parameters based upon passing an image through a trained neural network and using a descriptors layer of the trained neural network.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the disclosure, exemplary methods and/or materials are described below. In case of conflict, the patent specification, including definitions, will control. In addition, the materials, methods, and examples are illustrative only and are not intended to be necessarily limiting.

As will be appreciated by one skilled in the art, some embodiments of the present disclosure may be embodied as a system, method or computer program product. Accordingly, some embodiments of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, some embodiments of the present disclosure may take the form of a computer program product embodied in one or more computer readable medium(s) having computer readable program code embodied thereon. Implementation of the method and/or system of some embodiments of the disclosure can involve performing and/or completing selected tasks manually, automatically, or a combination thereof. Moreover, according to actual instrumentation and equipment of some embodiments of the method and/or system of the disclosure, several selected tasks could be implemented by hardware, by software or by firmware and/or by a combination thereof, e.g., using an operating system.

For example, hardware for performing selected tasks according to some embodiments of the disclosure could be implemented as a chip or a circuit. As software, selected tasks according to some embodiments of the invention could be implemented as a plurality of software instructions being executed by a computer using any suitable operating system. In an exemplary embodiment of the disclosure, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the disclosure. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present disclosure are described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as monitoring blood vessels in patients, might be expected to use completely different methods, e.g., making use of expert knowledge and/or the pattern recognition capabilities of the human brain, which would be vastly more efficient than manually going through the steps of the methods described herein.

BRIEF DESCRIPTION OF THE SEVERAL VIEWS OF THE DRAWINGS

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 is a graph showing probability of a vascular access thrombosis occurring within a 3-month period dependent on flow rate and on a change in flow rate, as reported by Besarab et al, "Access Monitoring is Worthwhile and Valuable", Blood Purification", February 2006;

FIG. 2 is a simplified illustration of a system for measuring blood vessels according to an example embodiment of the disclosure;

FIG. 3 is a simplified block diagram of a system for measuring blood vessels according to an example embodiment of the disclosure;

FIGS. 4A-4E are simplified flow chart illustrations of algorithms according to example embodiments of the disclosure;

FIGS. 5A and 5B are simplified illustrations of a pulse wave travelling along a vein;

FIG. 6 is a simplified flow chart illustration of a classifier method according to an example embodiment of the disclosure;

FIG. 7 is a simplified block diagram of a system for measuring blood vessels according to an example embodiment of the disclosure;

FIGS. 8A and 8B are images of optical components in a system constructed according to an example embodiment of the disclosure;

FIG. 9 is a simplified flow chart illustration of a segmentation method according to an example embodiment of the disclosure;

FIG. 10 is a simplified flow chart illustration of a registration method according to an example embodiment of the disclosure;

FIG. 11 is a simplified flow chart illustration of a method according to an example embodiment of the disclosure;

FIG. 12 is a simplified flow chart illustration of a method according to an example embodiment of the disclosure;

FIG. 13 is a simplified flow chart illustration of a method according to an example embodiment of the disclosure;

FIG. 14 is a simplified flow chart illustration of a classifier method according to an example embodiment of the disclosure;

FIGS. 15A-C show three different images of a same patient arm, according to an example embodiment of the disclosure;

FIG. 16A is a table showing a procedure for a medical person to examine a patient with reference to vascular stenotic lesions or thrombosis;

FIG. 16B is a simplified flow chart illustration of a method of examining a patient according to an example embodiment of the disclosure;

FIG. 17 is a simplified block diagram illustration of a method for examining a patient according to an example embodiment of the disclosure;

FIGS. 18A and 18B are images of a fistula of a patient taken at two different times;

FIGS. 19A and 19B are simplified drawings of a system for monitoring vascular access (VA) and/or fistulas according to two example embodiments of the disclosure;

FIG. 20 is an image of a system for monitoring vascular access (VA) and/or fistulas according to an example embodiment of the disclosure;

FIGS. 21A-C show three different images of a same patient's arm;

Figure 22A is a graph showing power spectrum of vibrations measured by analysis of images produced by laser speckle imaging;

FIG. 22B is a simplified flow chart illustration of a method for transforming data from a stream of images to a frequency spectrum according to an example embodiment of the disclosure;

FIG. 23 is a simplified flow chart illustration of a method for monitoring blood vessel functionality according to an example embodiment of the disclosure; and

FIG. 24 is a simplified flow chart illustration of a method for replacing a physical examination performed by medical staff for monitoring blood vessel functionality according to an example embodiment of the disclosure.

DESCRIPTION OF SPECIFIC EMBODIMENTS

The disclosure relates generally to the field of monitoring blood vessels in patients. Some aspects relate more particularly to early diagnosis of failure in blood vessel functionality, and even more particularly to early detection of failure of vascular access in patients undergoing hemodialysis treatments. Some aspects relate more particularly to measurements of fistulas.

Introduction

Monitoring by physical examination is cost-effective and a proven method to detect VA abnormalities. Unfortunately, nephrologists and HD staff generally have limited availability and are not well informed. As a result, regular physical examinations of VAs are not generally carried out in HD units.

Moreover, due to the complex nature of VAs, the surveillance strategies have failed to consistently detect stenosis under different scenarios. Although a low VA flow is associated with an increased risk of thrombosis, the association does not have adequate accuracy in predicting thrombosis. In contrast, VA flow and dynamic or static pressures surveillances were found to be inaccurate predictors of graft thrombosis and instead of preventing thrombosis yielded many unnecessary intervention procedures. Moreover, PTA induces a mechanical trauma, accompanying neointimal hyperplasia (NIH), risk of stenosis and impaired VA survival.

Both flow and pressure vary in patients during and, more importantly, between dialysis sessions. This makes each single measurement a potentially inaccurate predictor of stenosis, and therefore, also of thrombosis, and an evolving lesion may go unnoticed.

The hyperbolic relationship between flow and access pressures would be expected to occur within a given access if the outflow were the only source of stenosis. Unfortunately, this is not the case. Lesions in the inflow and within the body of the access do occur, and on average, the typical access has nearly two lesions/access locations at the time of referral. The lesions change the relationship between flow and pressure. Because of these confounders due to anatomic factors and the location of stenosis, there is little, if any, correlation between a single measurement of flow and pressure.

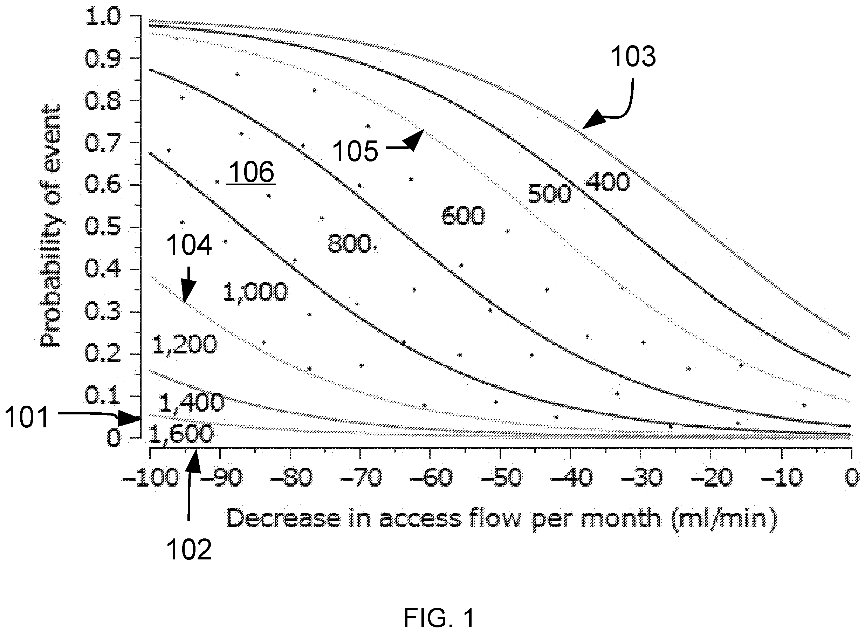

Reference is now made to Figure 1, which is a graph showing probability of a vascular access thrombosis occurring within a 3-month period dependent on flow rate and on a change in flow rate, as reported by Besarab et al, "Access Monitoring is Worthwhile and Valuable", Blood Purification", February 2006.

The graph of Figure 1 includes a Y-axis 101 showing probability of a vascular access thrombosis occurring within a 3-month period, various lines 103 showing flow rate in units of ml/min, and an X-axis 102 showing a change in flow rate per month, in units of ml/min.

Figure 1 shows that a probability of a vascular access thrombosis occurring within a 3-month period is dependent not only on the absolute flow at any time but also on a rate of change in the flow, if there is a change in flow (Besarab et al, "Access Monitoring is Worthwhile and Valuable", Blood Purification", February 2006).

An access with an initial flow of 600 ml/min and a 20-ml/min decrease in flow per month has a lower probability of thrombosis (22%) than an access with an initial flow of 1,200 ml/min and a decrease in flow of 100 ml/min (38%), even though the absolute flow is lower in the former (540 ml/min) than in the latter (900 ml/min) at the beginning of the observation period.

Thus, there is a need for monitoring solutions capable of detecting a forming stenosis early and predicting thrombosis, which overcome at least some of the following drawbacks of existing monitoring practices:

Poor compliance to routine VA physical examination by dialysis centers as outlined by guidelines;
An inherent inaccuracy related to a single-time physical examination or pressure/flow measurement of the VA;
An inherent inaccuracy of a single parameter such as flow or pressure;
Periodic measurements' results may be influenced by unrelated hemodynamic events; and
Measurement by different human caretakers may introduce inconsistencies.

Overview

An aspect of some embodiments of the present disclosure relates to replacing or adding to physical examination performed by medical staff/ nurses.

When a nurse or physician examines a patient's blood vessels, they typically use a three-step procedure: look, listen and feel.

The claimed invention is related to performing look, listen and feel by instruments measurements and computerized analysis.

The systems as described herein perform a look, listen and feel based on illuminating and imaging a patient's limb and analyzing the data collected from the imaging. In some embodiments, the systems teach how to predict fistula condition and potentially enable early prevention of failure.

The methods as described herein performs a look, listen and feel based on illuminating and imaging a patient's limb and analyzing the data collected from the imaging. In some embodiments, the systems teach how to predict fistula condition and potentially enable early prevention of failure.

In some embodiments blood flow is measured in a non-invasive manner, based on image processing of image of blood vessels in a human body. Physiological parameters which are known to affect vascular access (VA) are measured, and the measurements are optionally used to determine whether a patient should be scheduled for corrective procedure or proceed to undergo dialysis.

An aspect of some embodiments is related to performing feel, as described herein, by instruments measurements and computerized analysis.

In some embodiments, the listen as described herein is performed by instruments, optionally the same instruments.

In some embodiments, the look as described herein is performed by instruments, optionally the same instruments.

An aspect of some embodiments of the present disclosure relates to automatic detection and/or monitoring of an AV fistula in an images of blood vessels.

In some embodiments an image of blood vessels is analyzed, and a location where an artery is connected to a vein is determined to be a location of an AV fistula.

In some embodiments an image of blood vessels is analyzed, and a location where an artery appears to be connected to a vein is determined to be a location of an AV fistula.

In some embodiments an image of blood vessels is analyzed, and an AV fistula is measured to estimate geometric properties.

An aspect of some embodiments of the present disclosure relates to automatic, non-invasive measurement of parameters associated with blood flow.

In some embodiments the non-invasive measurement includes imaging blood vessels through skin, using reflected light and/or transmitted light.

In some embodiments, a probability of failure of vascular access is optionally estimated. In some embodiments the estimation is based on one or more of the parameters measured.

In some embodiments, a probability of occlusion formation is optionally estimated. In some embodiments the estimation is based on one or more of the parameters measured.

In some embodiments, a probability of thrombus formation is optionally estimated. In some embodiments the estimation is based on one or more of the parameters measured.

In some embodiments, a grade of stenosis is optionally estimated. In some embodiments the estimation is based on one or more of the parameters measured.

In some embodiments, a rate of stenosis formation is optionally estimated. In some embodiments the estimation is based on one or more of the parameters measured.

In some embodiments, a grade of VA maturation is optionally estimated. In some embodiments the estimation is based on one or more of the parameters measured.

In some embodiments, a rate of VA maturation is optionally estimated. In some embodiments the estimation is based on one or more of the parameters measured.

An aspect of some embodiments of the present disclosure relates to provide a visual report to a caregiver.

Attributes, one or more of which are related to some embodiments the disclosure, are listed below:

1. One or more of patient-related parameters, including images, are readily available for measurement(s) in a way that is potentially cost-effective and/or non-invasive (optionally, noncontact), and/or integrated into routine dialysis appointments.

2. An input to an algorithm described herein optionally includes one or more patient-related parameters in order to estimate probability of failure of vascular access, where each of the parameters can be available on a single-measurement basis or as multiple measurements along the time axis.

3. Some of the patient-related parameters are obtained using objective measurements, potentially not requiring high competence from a user, such as a patient and/or a health care professional.

4. Some of the patient-related parameters are optionally taken from the patient's specific medical record and include elements such as demographics (e.g. age, gender, weight and height), lab tests, imaging tests (e.g. X-ray, MRI) and results of a physical exam. It should be clear to a person skilled in the art that the parameters can be extracted in multiple ways, for example - directly typing exam results into a keyboard connected to a system as described herein, a computer process that accesses electronic medical records using a specific patient ID, speech-to-text conversion, voice recognition algorithms applied to verbal analysis of the staff and OCR of a printed/written documents.

Measurement of VA maturation: The VA has a unique tissue structure when compared with veins and arteries. The structure changes during a VA maturation process, and during a stenotic process.

Structural changes impact the mechanical and optical characteristics of the VA, thus monitoring of changes can potentially be measured, in some embodiments, by one or more of:

- Imaging: by way of a non-limiting example by measuring changes in contrast or intensity of reflected light and/or transmitted light;
- Non imaging: intensity of reflected light or transmitted light;
- Measurement of scattering and absorption coefficients (e.g. two distance steady state photon migration measurement).

For example, in some embodiments a system is configured to detect veins, monitoring VA during a maturation period potentially alters detection results. In an example of optical sensing, the response of the VA to light (one or more of transmission, reflection, absorption, scattering) potentially changes over the maturation period. Monitoring of maturation is potentially beneficial to raise a success rate of VA maturation by suggesting a timely pre-emptive correction. Measurement of blood vessel layers, or a ratio between blood vessel layers or the changes in ratio between layers or changes in absolute values of layers during maturation or stenotic process.

Accuracy of estimating maturation (maturity level, stage, rate, completion) or probability of failure of vascular access, occlusion formation and probability of thrombus may be improved by using one or more parameters generated from non-invasive measurement. The parameters used can be directly measured or be a result of a pre-processing applied on the measurement. Such pre-processing can be application of various algorithms as well as combination of several parameters and utilization of multiple measurements over time.

Examples for metrics or phenomena that are optionally extracted and used in some embodiments of the disclosure:

1. Pulse wave velocity - In some embodiments detect reflection or absorption of optical radiation from at least two points in an image frame. In some embodiments changes in electrical impedance as measured by electrodes placed between and/or along the two points, along the blood vessel or tissue area. Optionally, the two points include sections known to be more susceptible to develop stenosis. More generally, at least one point is used for measuring pulse wave shape (such as, for example, pulse wave amplitude, Full-Width Half Max (FWHM)).

In some embodiments pulse wave amplitude is optionally measured. An optional method for measuring pulse wave amplitude includes measuring a first measurement of an area of a location along a vein identified as a widening of a blood vessel due to a pulse wave. An area of the same location in a different image, when the pulse wave is not at that location, is also measured in a second measurement. A difference between the first measurement and the second measurement is optionally associated with the pulse wave amplitude. In some embodiments the pulse wave amplitude is taken as a feature which corresponds to mechanical properties of a vein all, and/or with maturity of an AV through which the pulse wave travels.

In some embodiments a Pulse Wave Analysis (PWA) is optionally performed to assess variance related to vascular stiffness which is associated with additional risk factors such as cardiovascular disease or atherosclerosis which in turn - may impact viability over time of the VA. A quality of the pulse is optionally scored, and changes over time and between different sections are optionally included in the analysis, in some embodiments.

2. Appearance and development of collateral veins and their characteristics, such as: density, size, distance from the VA, orientation, filling etc. by image processing and/or other detection methods, e.g. measure contrast - by absorption of light in the visible or NIR wavelength; or emission at the far IR wavelength. Other measurement options include measuring an amount of change of absorption in the visible and near IR and amount of emission in the far IR. Another optional way to measure development of collaterals is measuring temperature changes of the VA surrounding. In some embodiments detection of appearance and development of collateral veins optionally uses reference images or measurements taken from a prior examination. In some embodiments trend analysis of collateral vein development rate optionally uses frequent examinations. The examinations are optionally performed daily, every dialysis session, every week, bi-weekly, or monthly.

In some embodiments collateral veins are detected by comparing a new image to a previous image and counting veins - an increase in the number of veins is optionally taken to mean that the new veins are collateral veins.

In some embodiments, appearance and/or development of collateral veins is detected by extracting features from one image or measurement.

Rationale: detection of a collateral vessel potentially indicates a flow limiting (hemodynamic significant) lesion. Collateral vessels may develop and enlarge, dissipating the increased intra-access pressures in the setting of outflow stenosis.

3. A blood vessel's smallest diameter by image processing (stenosis location).

4. Detection of a point of narrowing by estimation of mechanical reflection waves or changes in local pressure/flow for example by measurement of electrical impedance changes.

5. A blood vessel's largest diameter by image processing (appearance and size of aneurysms).

6. Detection of vessel collapse when arm or leg is elevated.

7. Using Near Infrared (NIR) (700-1000nm) reflected and/or transferred spectroscopy for measuring amounts of oxygenated and deoxygenated hemoglobin (Hb).

8. Spectroscopy analysis for oxygenated and deoxygenated hemoglobin (Hb)

9. Audible sound of the VA (bruit).

10. Palpated pulsation of the VA (thrill).

11. Analysis of electrical impedance changes at VA using signal processing methods known in the art.

12. In some embodiments in which multiple measurements of the same parameter are taken over time, the measurements may be synched according to a detected breathing cycle and categorized for the detection algorithm in respect to their relative time along the breathing cycle. Such synching and categorization are potentially beneficial, for example, when evaluating changes in the oxygen mix over time, but can also improve accuracy of other measurements, such as pulse wave velocity.

Output of a system as described herein may be in the form of an audible alarm, visual alarm, image, sequence of images, or a video providing the medical personnel guidance for fast and accurate intervention (e.g. give a recommendation to the medical personnel regarding the best location(s) for intervention). The system may recommend treatment for a patient (PTA, not to intervene, thrombectomy). The recommendation is optionally based on information collected by the system.

According to an aspect of some embodiments of the present disclosure, output of the system during a test is optionally analyzed and/or optionally used to guide a patient through a test in order to perform the test correctly. By way of a non-limiting example, in an elevation test - verifying that the elevation/position of a limb is correct. In some embodiments, there is also an alert to a nurse/technician in case a patient has not performed the test correctly or requires help.

In some embodiments, the above-mentioned output is optionally used to support a remote physical examination to be performed by a patient while the system provides feedback on correct performance of the examination and/or alerts remote support personnel, such as a nurse or technician that additional guidance is required.

In some embodiments, system output is optionally provided differently to different consumers of the data. For example: a dialysis nurse is provided with a general interpretation on a likelihood of clinically meaningful stenosis formation and an interventional radiologist is provided with an alert with an annotated image and/or a report highlighting parameters such as location, severity and rate of stenosis formation.

According to an aspect of some embodiments of the present disclosure there is provided a system and methods for measuring parameter related to fistulas.

In some embodiments, there is provided a system which includes optical apparatus to acquire one or more images of the same patient's fistula along a surveillance period.

In some embodiments, one or more measurements and/or features are extracted from the image(s) - and their changes over time are monitored. In some embodiments, the features are timeline derivatives of parameters measured or estimated in the image(s), by way of a non-limiting example changes in number, branching & size of collateral veins happening over a period of time, such as days/weeks/months.

In some embodiments, using a machine-learning-derived method to identify a pattern within the above changes which may potentially lead to a significant clinical end point (e.g. Stenosis of the fistula) before there are clinical signs or symptoms which human nurses can identify.

In some embodiments, a system is provided which measures parameters relating to a fistula by optical means.

In some embodiments, structured light is projected onto a patient's body or limb, and the body is imaged. In some embodiments, the structured light may include horizontal and/or vertical stripes of equal or different widths and/or various light patterns other than stripes.

In some embodiments, imaging the structured light is used to provide information about an extent of the fistula, for example length of a long axis of the fistula along the body: breadth of a short axis of the fistula along the body: shape of the fistula as it appears in the image: segmentation of the fistula circumference, eccentricity index and/or aspect ratio of each segment, smoothness and/or roughness of a fistula outline
In some embodiments, structured light patterns are projected onto a patient's body or limb, and the body is imaged, providing information about a three-dimensional shape of the fistula or organ.

In some embodiments, the system identifies changes in the shape of the fistula and/or an organ near the fistula. In some embodiments, a projector is used to project one or more light patterns (e.g. structured light). In some embodiments, a method measures and/or estimates how the patterns deform on a patient's organ to measure the organ's shape and shape changes over time.

In some embodiments, structured light patterns are projected onto a patient's body or limb, and the body is imaged, providing information about a three-dimensional shape of the fistula, by way of some non-limiting examples volume of an entire fistula or segments of a fistula (e.g. needle insertion points); characteristics and/or variance of curvature; changes in shape and/or volume of an underlying arm/organ section near a fistula; and three-dimensional surface features such as smoothness and/or roughness.

In some embodiments, Laser Speckle Interferometry (LSI) is used. In some embodiments, LSI is used to record and look at vibrations of the fistula surface that correlate with the blood flow and turbulence inside. Changes in the blood flow and turbulence are typically correlated with stenosis events, and potential development of clinical conditions.

In some embodiments, imaging the speckled light is used to provide information about dynamic effects in the fistula, for example heart pulse, blood flow turbulence, and optionally produce spectrograms of vibrations of a fistula.

In some embodiments, images of the body are taken some period of time apart, and differences between the images are optionally used to determine differences in the shape of the fistula.

In some embodiments, the images are taken days, weeks, months or years apart, and differences between the images is optionally used to measure and/or monitor changes in size or shape of the fistula.

In some embodiments, the images are taken seconds or minutes apart, for example with a limb such as a hand held horizontally followed by the hand held vertically, and differences between the images is used to measure and/or monitor one or more of: whether at least some of the blood in the fistula can evacuate the fistula; a rate of blood evacuation; a degree of blood evacuation from the fistula and/or specific portions of the fistula; and collapse of one or more needle insertion points.

In some embodiments, the images are taken fractions of a second apart, as a video clip or movie, and differences between image frames is optionally used to measure and/or monitor dynamic parameter related to the fistula, such as heart pulse, blood flow turbulence, and optionally produce spectrograms of vibrations of a fistula.

In some embodiments, the spectrogram is optionally produced by selecting one or more pixels in the image frames which show a large or even a maximal variation of intensity over time. In some embodiments, the number of pixels selected is optionally in a range of 1-100 pixels. In some embodiments, the values of pixel intensity of this or these pixels are used to compute a function of light intensity over time. In some embodiments, a frequency spectrum of the light intensity is optionally produced by transforming from the time domain to the frequency domain, for example by a Fast Fourier Transform (FFT).

In some embodiments, an analysis is made of changes in the dynamic parameters relate to the fistula between imaging sessions, to monitor changes in the fistula and the patient's conditions.

In some embodiments, performing the above together with Near IR imaging potentially enables collecting data that correlates with examinations required to be perform by nurses and/or physicians and that is already clinically proven to have predictive value to identify stenosis events.

According to an aspect of some embodiments of the present disclosure there is provided a system and methods for implementing and recording more than one technique or modality, for example one or more of structured light; laser speckle interferometry; image analysis and Near IR imaging modalities, using one imaging device.

In some embodiments, the system includes a processor and an imaging device which includes a Digital Light Processing (DLP) projector and a Near IR camera.

According to an aspect of some embodiments of the present disclosure there are provided systems and methods for analyzing vibrations of light reflected from a patient's body.

In some embodiments, pulsatility of a heart is monitored.

In some embodiment, analyzing the pattern of vibrations caused by flow through or in vicinity to the fistula optionally detects full or partial occlusions of either the inflow or outflow pathways.

In some embodiment, analyzing the pattern of vibrations caused by flow through or in vicinity to the fistula while imposing local pressure to either inflow or outflow pathways optionally detects full or partial occlusions of either the inflow or outflow pathways.

In some embodiments, analyzing the vibrations optionally detects onset of flow through the fistula related to normal heart activity, (the diastole or systole phases of the heart cycle).

In some embodiments, analyzing the vibrations optionally detects onset of flow through the fistula related to sudden release, (partially or full collapse or expansion of the fistula).

In some embodiments, analyzing the vibrations optionally detects a period of inflow of blood to a fistula, followed by a sudden opening of an obstacle which enables blood to flow out of the fistula. Such opening may happen during high pressure of a heart systole. In some instances the sudden opening is called hammering. In some embodiments, the hammering is detected by measuring amplitude of vibrations, optionally relative to the amplitude at other times, for example other times during a heartbeat.

In some embodiments, analyzing the vibrations related to onset of flow, for any or all types of onset, optionally measures a parameter value or a change in parameter value or a change in a characteristic parameter value, or a variance of the parameter value. The parameters may be one or more of: Intensity, Energy, Steepness of onset (derivative of value), Relaxation time, Temporal-width, Duty-cycle, Spectral-content, Spectral-width, or any combination of such.

In some embodiments, analyzing the vibrations related to onset of flow optionally measures a parameter value related to the time-delay or phase-delay between onsets related to sudden release, and onsets related to normal heart activity.

In some embodiments, analyzing the vibrations related to onset of flow optionally measures a parameter value related to the regularity or self-similarity of a series of onsets of the same source.

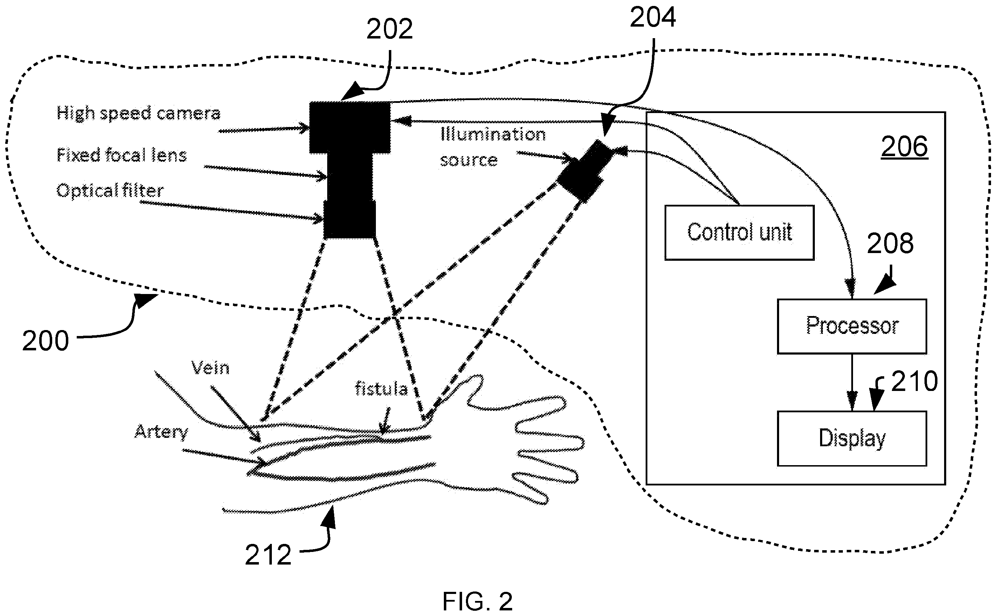

Reference is now made to Figure 2, which is a simplified illustration of a system for measuring blood vessels according to the claimed invention.

Figure 2 shows a top level set up configuration of an exemplary system 200 for measuring blood vessels.

In some embodiments the system 200 includes at least one illumination source 202 and at least one detector 204, such as a camera.

In some embodiments the system 200 further includes a control unit 206, which activates the illumination source 202 and the camera 204, and a processor 208, which receives and analyzes images generated by the camera 202.

In some embodiments, the generated images and/or the data generated following the analysis of the images may be displayed on an optional display 210 coupled to the processor 208, either wirelessly or via a wired connection.

In some embodiments, the processor 208 and the display 210 may be implemented in a single device, such as a laptop, tablet or smartphone. In some embodiments, a scan system is applied that moves the detection unit (automatically or manually) and scans an organ at more than one point. Figure 2 describes the system 200 applied to an arm 212.

The system is capable of implementation with other organs, without limitation.

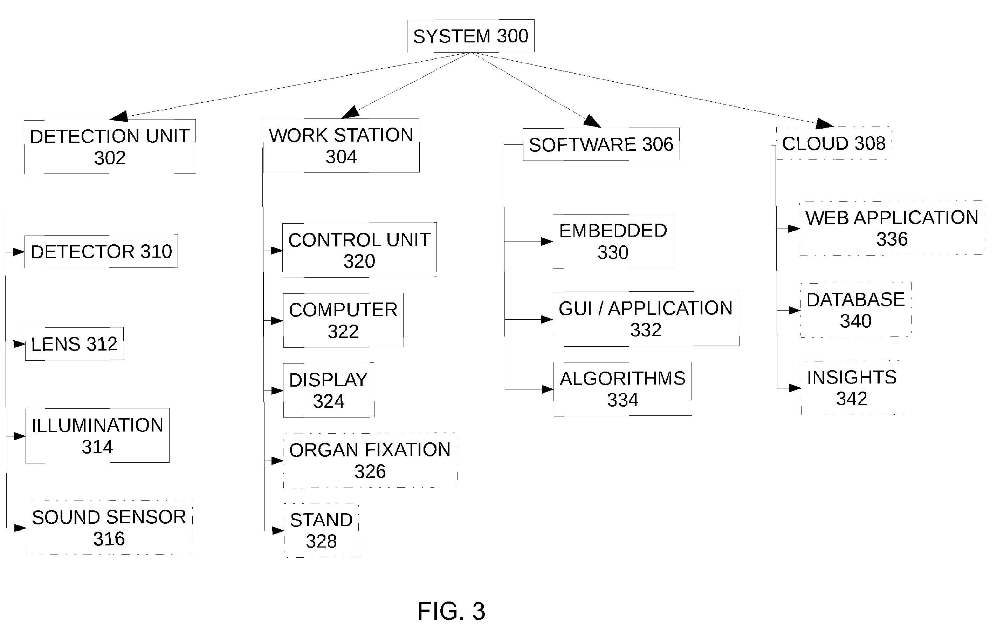

Reference is now made to Figure 3, which is a simplified block diagram of a system for measuring blood vessels according to an example embodiment of the disclosure

Figure 3 describes the top-level block diagram of an exemplary system.

In some embodiments the system includes at least two main units; a detection unit 302 and a software unit 306.

The system may include additional units, such as a work station 304, optional cloud infrastructure 308, etc.

In some embodiments, the software unit 306 includes at least two sub-units, an embedded unit 330 and an algorithms unit 334. The software unit 306 may include additional blocks, such as a Graphical User Interface (GUI) unit 332, etc.

## Detection unit

In some embodiments, the detection unit 302 uses:

1. visual/optical detection, to acquire images containing information to be further analyzed.
2. Speckle imaging - When an object is illuminated by laser light, the backscattered light forms an interference pattern consisting of dark and bright areas. This pattern is called a speckle pattern. If the illuminated object is static, the speckle pattern is stationary. When there is movement in the object, such as red blood cells in a tissue, the speckle pattern will change over time. The speckled images contain information related to changes in the blood vessels which is analyzed and extracted by image processing.
3. Dark field \ side illumination -

   a. Specular reflections not reaching the camera
   b. Only diffused scattering rays are captured by the camera
   c. Reducing surface reflection
   d. Contrast profile changes with changing the angle between light source and detector.

4. Transmitted illumination - Illuminates the back surface of a sample. The sample is placed between the illumination source and the sensor device. Transmitted illumination potentially improves the image contrast and/or potentially increases the depth at which blood vessel can be imaged.
5. Photo acoustic imaging potentially enhances contrast between different mediums because of differences in changes in the optical characteristic of the different mediums. Photo acoustic imaging potentially reduces scattering in tissue because of averaging of the refraction index gradient in tissue components, potentially resulting in a greater penetration depth of light.

In some embodiments the detection unit 302 includes one or more of the following components:

1. One or more detectors/sensors/cameras 310 (e.g., CCD or CMOS, InGaAs sensor, micro bolometer), which are sensitive to one or more of visible, near infrared light, short-wave infrared (SWIR) light. In some embodiments a sensor frame rate can range between single-frame to a high frame rate. Sensor frame rate are in a range of, for example, 5, 10, 16, 24, 30, 50, 60, 100, 165, 200, and even up to 300-frames per second (fps).
2. One or more lenses 312 (zoom or fixed focal length) and/or filters
3. One or more illuminators 314 or emitters (e.g., an illumination source that can be coherent or non-coherent, narrow spectra or broadband, UV, visible, SWIR, far IR, NIR - for example NIR led or green (532nm) laser). Emitters can be coaxial or in different angles relative to the detector 310 and a VA.

The operation mode can be stills or video.

4. One or more polarization filters (elliptical and/or linear)
5. One or more optical bandpass filters
6. The detection unit optionally includes a scan system or a moving bar scanner.

In some embodiments, the detection unit 302 optionally uses an audio/sound detection sensor 316, instead of, or in addition to, visual/optical detection, and the detection unit 302 may optionally include one or more audio sensors.
In some embodiments, the detection unit 302 may include vital signs sensors.

## Software unit

In some embodiments, the software unit 306 includes one or more of the following components:

1. GUI - graphic user interface / Application 332 for one or more of: operating a test procedure, displaying images and/or results and/or inserting or importing patient clinical information.
2. Embedded 330 - for controlling the detection unit 302.
3. Algorithms unit 334 - the algorithms unit includes algorithms, or software modules, for:

   Image processing
   Machine learning (ML)

In some embodiments, inputs for the ML algorithm are images and/or data captured by the detection unit 302.
In some embodiments, the inputs may include also clinical information of the patient and/or vital signs.
In some embodiments, the work station 304 optionally includes a computer, a screen, a keyboard, one or more knob controls, a mechanical interface for the imaging unit, and an electric power supply or interface to electric power. In some embodiments, the work station 304 may also include an "organ fixation surface".
In some embodiments, the work station 304 optionally includes one or more of:

   a control unit 320, for controlling operation of the detection unit 302 and/or one or more of the components of the detection unit 302;
   a computer 320;
   a display 324;
   an optional organ fixation surface or device 326, for placing an organ at a specific location relative to the illumination 314 and/or the detector 310; and

a stand 328, for placing components of the system at a specific location relative to a patient's organ.

In some embodiments, the cloud infrastructure 308 optionally includes one or more of the following cloud services

a storage (database) server 340;

a Web application server 336;

a computing service for machine learning, such as refining algorithm(s) based on new data; and/or for analytics - to provide measures of function and metrics to a user; and/or insight - to provide metrics related to current or a predicted future clinical condition of the VA.

A machine learning algorithm - may be supervised or unsupervised, learning based on database of images and/or of patient parameters produced by an embodiment of the disclosure, and/or of meta data such as a patient's, disease, vital signs, parameters from a dialysis machine and/or other data available in a medical electronic record, optionally including previous interventions for this patient, additional risk factors, comorbidities, and so on.

The steps include one or more of:

1. Feature extraction from the images
2. Trend calculation of the features
3. Running ML on the feature vectors and/or on the features vector trends.

In some embodiments an outcome of the ML is a statistical classifier model that distinguishes between less or more than 50% AV patency.

In some embodiments Analytics and Insight run on the metadata and patient records, and calculate statistics of failure of the AV based on the patient profile (metadata and medical health record).

In some embodiments analytics is optionally performed on a clinic's performance, for example how many stenosis events per year.

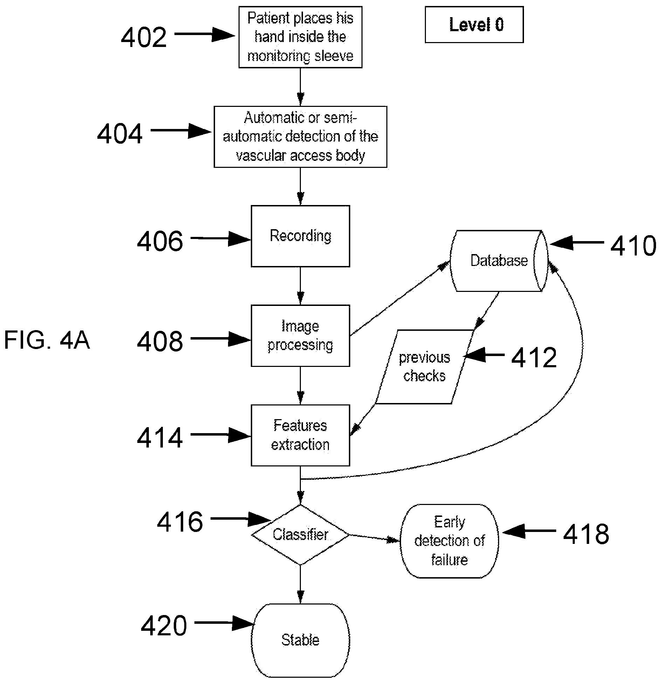

Reference is now made to Figures 4A-4E, which are simplified flow chart illustrations of algorithms according to example embodiments of the disclosure.

Figures 4A-4E show flow charts depicting exemplary algorithms which may be implemented, by way of a non-limiting example, in the system's software unit 306 or in the cloud unit 308.

Figure 4A illustrates a procedure flow. Figure 4A illustrates the procedure flow on a vascular access, as an example.

First, a subject's organ (e.g. arm) is placed inside a fixating sleeve (402), under the detection unit. In some embodiments, the organ is an arm or leg, and all measurements are taken when the organ is approximately perpendicular to the ground (pointing up or down). In some embodiments, some of the measurements are taken when the organ is approximately parallel to the ground, and some measurements are taken when the organ is perpendicular to the ground (pointing up or down). In some embodiments, some of the measurements are taken when the organ is lower than the patient's heart, and some measurements are taken when the organ is higher than the patient's heart.

Next, a region of interest (ROI) is detected (404). In some embodiments, the ROI is the vascular access body and/or surroundings of the vascular access body. The detection can be done either automatically by the system or manually by a physician/user.

A next step is taking one or more measurements (406), e.g. images, of the ROI.

The images go through a processing algorithm (408), e.g., image processing algorithm, and are then optionally saved into a database 410).

A next step is to extract features (414) from the current examination measurements, e.g., images, and from the previous examination measurements (412), e.g. images.

The features are sent to a statistical model which may classify (416) between "Early detection failure" (418) and "Stable" state (420) of the vascular access body.

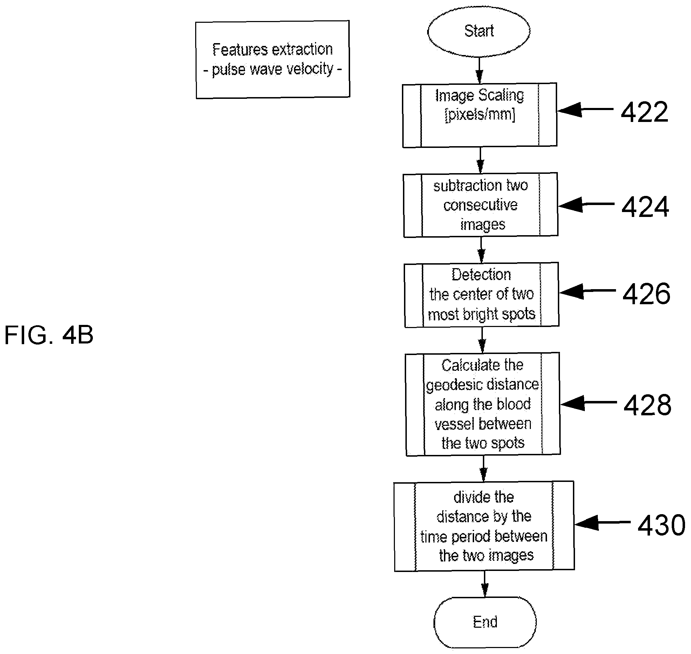

Figure 4B illustrates an exemplary algorithm flow of extracting features of the "pulse wave velocity" phenomena.

A first step is pre-processing (422), e.g., to detect the image scale, for example in units of mm.

A second step is to subtract the first image from the second (424). The result includes two bright spots.

A next step is to detect the centers of the bright spots (426) and to calculate a distance along a path along the blood vessel between the centers of the bright spots (428).

A next step is dividing the calculated path by the time period between the two images (430), producing a result of a pulse wave velocity.

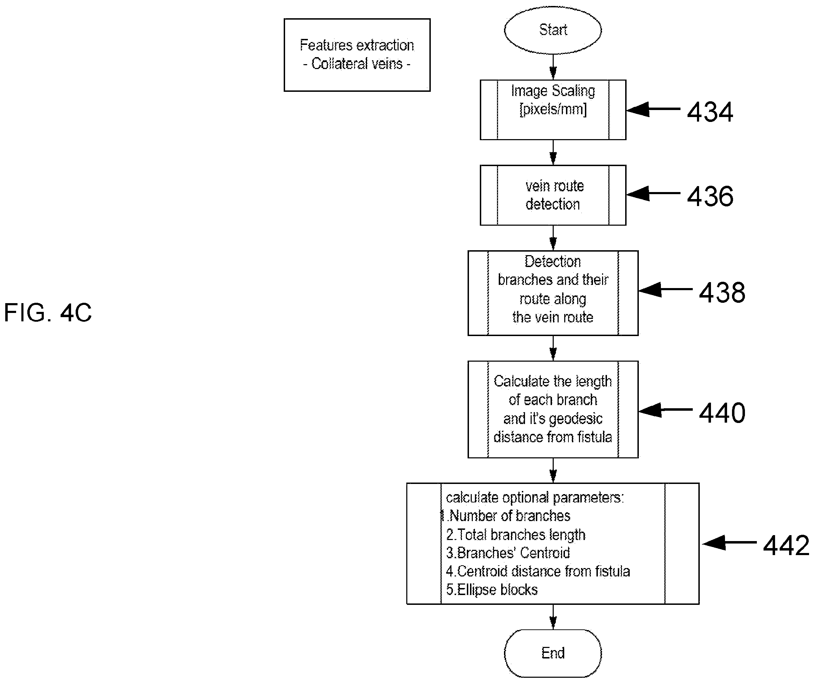

Figure 4C shows an exemplary algorithm flow of extracting features of the collateral veins phenomena.

A first step is pre-processing (434), e.g., to detect the image scale, for example in units of mm.

A second step is to detect the vessel's route and/or branches (436).

A next step is to calculate the length of each branch and its distance, along a vein route, from a fistula (438).

The above method is also described in more detail below, under the heading of "Detection of vascular access (VA) body algorithm" and in several location where segmentation is described.

A further step includes calculating parameters that describe the collateral veins phenomena (442), including one or more parameters such as:

1. A number of branches.
2. Total branches' length.
3. Branches' centroid.
4. Centroid distance from the fistula.
5. Ellipse blocks.
6. Diameter of branches, optionally detecting blood filling the lumen of a vessel.

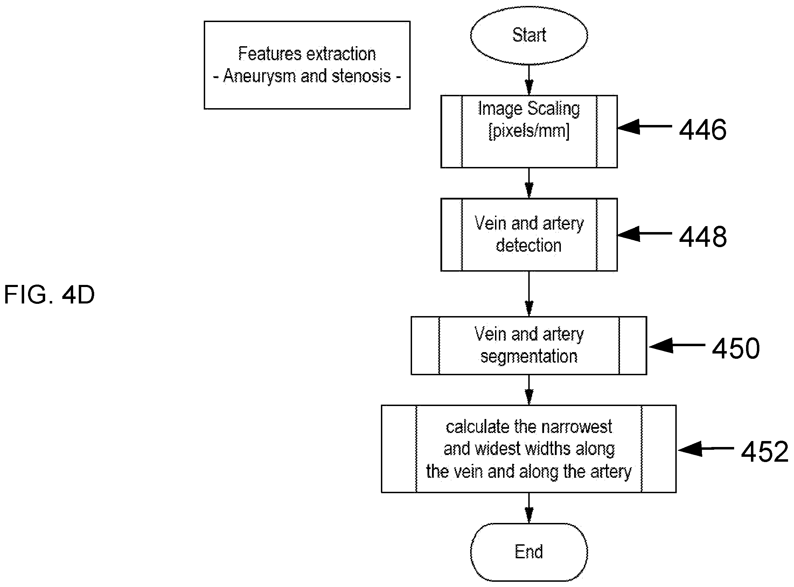

Figure 4D shows an exemplary algorithm flow of extracting features of the aneurysm and stenosis phenomena.

A first step is pre-processing (446), e.g. to detect the image scale, for example in units if mm.

A next step is to detect the vein and/or artery route (448).

A next step is to and segment the vein and/or artery route (450).

A next step is to find and calculate the narrowest and widest widths along the vein and/or artery routes (452).

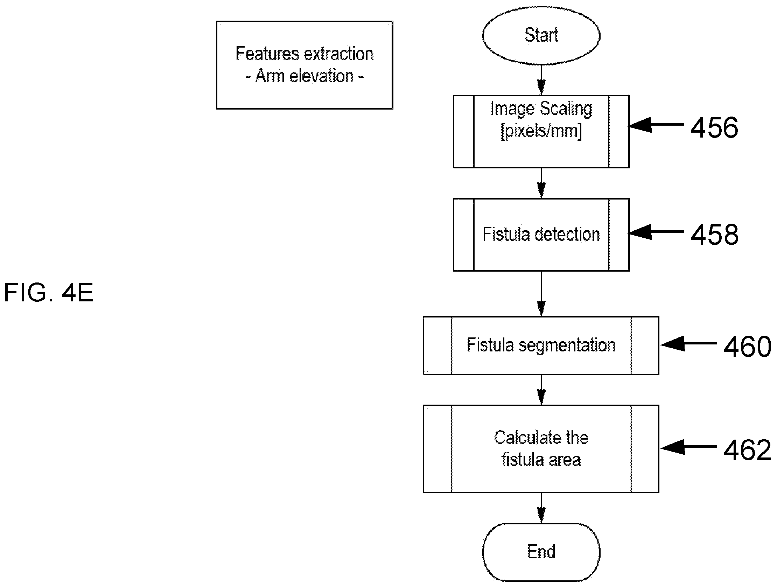

Figure 4E shows an exemplary algorithm flow of extracting features of an arm elevation examination. It is to be understood that the algorithm flow is applicable to other subject organs, and it is not limited to the arm.

In some embodiments two images are obtained after an arm is elevated, to track changes in outflow, which translate to changes, over a short period of time, in the volume of a fistula. In a normal outflow state - the fistula contents "quickly" (over a few seconds) emptied, and a difference in shape/area between the two images is detected and/or measured. In an obstructed outflow state - the fistula contents do not evacuate fast enough, and a smaller change, if at all, is detected/measured in the shape/area of the fistula. Tracking such changes over time - enables tracking changes in patency of a fistula.

In some embodiments, arm elevation examination is applied when the arm is elevated (pointing up or pointing down, perpendicular to the ground, and/or above the heart level).

In some embodiments, A first step is pre-processing (456), e.g., to detect the image scale, for example in units of mm.

A next step is to detect the vascular access (fistula) in the image (458).

A next step is to segment the vascular access (fistula) in the image (460), optionally segmenting the fistula from other portions of the image.

A next step is to calculate the vascular access area in the image (462).

In some embodiments, the arm elevation examination starts by taking a first image when the arm is parallel to the ground, and a second image when the arm is perpendicular to the ground (pointing up or down) above heart level.

After obtaining the first image and the second image, a next step is pre-processing, e.g., to detect the image scale, for example in units of mm, in both images.

A next step is to detect the vascular access in both images.

A next step is to calculate the vascular access area in both images.

A next step is to subtract the first vascular access area from the second vascular access area.

In some embodiment, the arm elevation examination starts by moving the arm (or any other subject organ) from a first position, where the arm is approximately parallel to the ground, to a second position, where the arm is approximately perpendicular to the ground (pointing up or down).

A next step is taking two images of the elevated arm.

A next step is pre-processing, e.g., to detect the image scale, for example in units of mm, in both images.

A next step is detecting the vascular access in both images.

A next step is to calculate the vascular access area in both images.

A next step is subtracting the first vascular access area from the second vascular access area.

A next step is dividing the calculated difference by the time period between the two images.

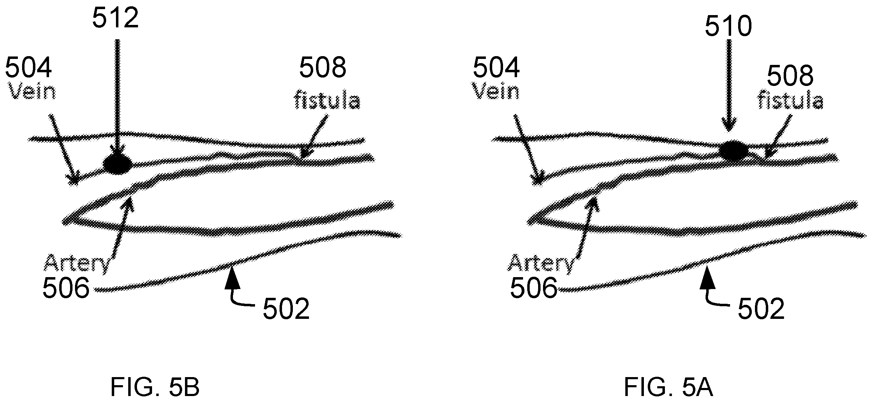

Reference is now made to Figures 5A and 5B, which are simplified illustrations of a pulse wave travelling along a vein.

Figure 5A shows a first image and Figure 5B shows a second image, taken a short time later.

The images of Figures 5A and 5B show an arm 502, a vein 504, an artery 506, and a fistula 508 where the vein 504 is connected to the artery 506.

Figure 5A shows a first location 510 where the vein is enlarged by pressure of a pulse wave, at a time $t_0$.

Figure 5A shows a second location 512 where the vein is enlarged by pressure of the pulse wave, at a time $t_1$.

The second location 512 is further along the vein 504 relative to the first location 510.

Pulse wave velocity is optionally measured by measuring a distance between the first location 510 and the second location 512, divided by a time difference between the capture of the first image and the second image.

In some embodiments the time difference is a fraction of a second. By way of a non-limiting example, when an image frame is approximately 15-30 centimeters wide, imaging a pressure wave progressing along a blood vessel is optionally done at a frame rate above 120 fps, for example at 165 fps.

In some embodiments, in order to have both of the above-mentioned locations with an image frame with a field of view of 160mm, and for a pulsed wave velocity of approximately 20m/s, the time different is <6ms. Such a time difference applies to all pulse wave velocities that are smaller than 20m/s.

One or more of the following algorithms may be implemented in the system described herein:

Predictor / classifier methods

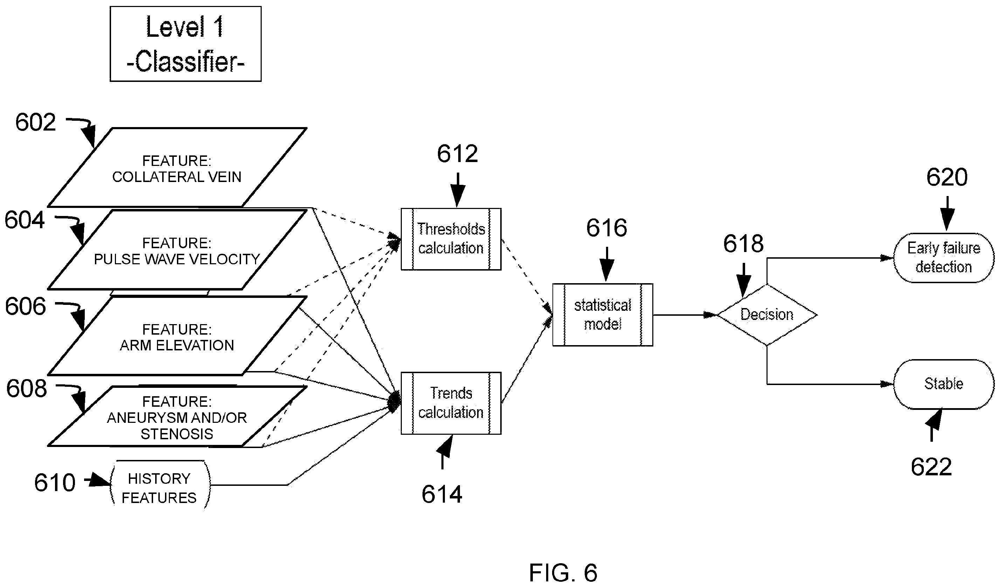

Reference is now made to Figure 6, which is a simplified flow chart illustration of a classifier method according to an example embodiment of the disclosure.

Figure 6 shows input of one or more feature descriptors, such as: a collateral vein descriptor 602, a pulse wave velocity descriptor 604, an arm elevation descriptor 606, and an aneurysm and/or stenosis descriptor 608.

In some embodiments the inputs 602 604 606 608 are fed into a threshold calculation unit 612.

In some embodiments a trends calculation unit 614 optionally accepts input of a historical and/or trend descriptor 610, optionally from a local or a remote database.

In some embodiments the trend calculation unit 614 produces a trend data output.

In some embodiments the threshold calculation unit 612 produces a threshold data output.

In some embodiments one or more of the above outputs are fed into a statistical unit 616.

In some embodiments output of the statistical unit 616 is input to a decision unit 618.

The decision unit 618 optionally produces a decision that the VA is determined to be "stable" 622, or that a failure is detected 620.

Figure 6 describes an exemplary classifier algorithm, which may be based on machine learning (supervised or non-supervised) tools or on heuristic rules that execute the following steps:

1. Data analysis, such as image processing.
2. Extracting features from one image or a set of sequential images of collateral vessels development, VA zone, etc.

Some examples of features include: smallest radius size of body of VA, pulse wave velocity, collateral veins sizes and density, distance of collateral veins from the AV or fistula. etc.

The features can also be a variation of the features between sequential images and/or a rate of variation of the features between sequential images.

3. Classifying the condition of the VA based on the extracted features. The classification can be base rule, threshold and/or statistical model. A statistical model can be based on a machine learning algorithm, such as: SVM (Support vector machine), logistic regression, neural network, decision tree, decision forest, "k means", etc.

The classification can be between two levels (intervention needed or not) or between more than two levels.

A non-limiting example of methods used for predicting and/or classifying include one or more of:

A table of parameter values;

Regression of parameter values associated with a patient;
K Nearest Neighbors (KNN) as applied to parameter values;
Support Vector Machine (SVM);
Deep learning;
Neural Network(s).

In some embodiments, machine learning uses a training set. A non-limiting example of producing and using a training dataset includes measuring parameters as described herein, for N patients, repeatedly over a period of time.

During the period of time, recording which patients had blood vessel failures, and/or which patients were examined by additional techniques such as human examination, ultrasonography, X-ray imaging, and what the additional techniques' determination was. The parameters and the determination potentially produce a training dataset, which can be used to train the above-mentioned machine learning methods, or to produce KNN dataset.

In some embodiments, some or all of the parameters measured for the datasets described above are also measured for patients for prediction and/or classification purposes.

It is noted that parameters which are collected over time optionally include measured values and parameters calculated from measured values, first derivatives of the parameter values, and second derivatives of the parameter values.

It is noted that a nurse or physician performing a blood vessel test by the look, listen and feel method typically provide a decision or classification based on a value of one or two parameters, while systems and methods as described herein potentially use more parameters, and potentially arrive at more accurate decisions or classifications which are based on more data of the patient and/or of a group of patients used for producing the training set.

Scaling algorithm

A scaling algorithm calculates the image scale (for example scaling pixels to mm). The scaling may be used for calculating absolute or relative values of one or more of a vessel's radius, pulse wave velocity, size of collateral vessels, density of collateral vessels, and distance of collateral vessel from VA.

Registration algorithm

A registration algorithm may perform automatic or semi-automatic registration between two or more sequential images.

The registration algorithm may align and/or scale two or more images that contain the same object in different positions or angles of view or different fields of view.

In some embodiments inputs to the registration algorithm include at least two images and in case of semi-automatic registration, optionally, one or more points that are marked by the user on the two images.

The registration algorithm potentially enables the system to measure a variation between at least two examinations, no matter how the arm, or another examined organ, is positioned during the different examinations.

In some embodiments, registration of at least two images of the same patient that contain a VA object is optionally done by detection (e.g. segmentation) of the VA and fitting the VA image in a first image by geometrical transformation to the VA image in a second image.

Detection of vascular access (VA) body algorithm

In some embodiments automatic or semi-automatic detection of the vascular access body location in the image is performed.

In some embodiments input for an algorithm for detection of a vascular access body includes at least one image that contains the vascular access body in the image frame.

In some embodiments an optional input is a set of one or more points along a blood vessel which includes the VA body, optionally marked by a physician/nurse on an image which includes the VA body.

The algorithm output may be a set of the vascular access body pixels in the image.

In some embodiments computerized detection of the VA body is based on a unique VA shape, size, orientation, position and etc.

In some embodiments, a device such as, by way of a non-limiting example, an "ELY-1000 vascular imaging instrument for Arterial puncture" as developed by ELYNNSH MEDICAL, is used. The device, according to the manufacturer, assists medical staff in identifying subcutaneous arteries during an arterial puncture, and can conveniently & quickly display the exact location of the arteries and direction.

In some embodiments a location is detected in an image, where an artery and a vein are connected or appear to join.

In some embodiments, a blood vessel providing blood to a VA is elevated by surgery toward the skin surface. Because of depth differences of blood vessel segments, an image which cover a field-of-view (FOV) which includes a VA, the VA often appears as a closed contour centroid. Tissues surrounding the VA body are often deeper under the skin than the VA body.

In some embodiments, the difference in depth is optionally detected by the VA body potentially showing up as a darker area than native or surrounding vessels. For example, when NIR illumination is used, the NIR light is absorb in the blood Hgb, and blood vessels closer to the surface appear darker than deeper vessels.

Pulse wave velocity algorithm

Reference is again made to Figures 5A and 5B, which describe an exemplary method for measurement of pulse wave velocity.

Pulse wave velocity is also a common indicator of blood vessel stiffness and can be obtained by measurement of the distance and the pulse wave transit time between two points of vessels. Pulse wave velocity can be measured locally, regionally or systemically.

The term locally is used to mean along a fistula and nearby related vessel structures.

Physiologically, there is a relation between the pulse velocity, blood flow and pressure inside the vessel.

The pulse wave (caused by heartbeat) travels from the heart to the arteries, and from the veins back to the heart. When the pulse is traveling, it temporarily deforms the blood vessel (e.g., the vein) at a moving discrete point and time.

For example, the vein radius may temporarily expand at a certain point along the vein. This point can be detected by measuring the absorption of light by the blood flowing in the vein - the location of the expanded vein shows as a darker or a lighter point along the vein (depending on a method of measurement, such as reflection or transmission).

By detecting points associated with an expanded blood vessel in two or more sequential images, while the time between capture of the images is known, pulse wave velocity can be calculated. Pulse wave velocity equals the distance between two points divided by the time between capture of the two images.

Example embodiment - system description

The system may measure one or more of the following example phenomena: vessel diameter, pulse wave velocity, NIR (e.g. 700-1000nm) reflected spectroscopy, appearance of collateral veins and their characteristics, such as: density, size, distance from the vascular access and oxygen concentration at the vascular access.

In some embodiments the NIR spectral range is used for blood vessel imaging. A spectral window exists from approximately 700 nm to approximately 900 nm, where light can penetrate deep into tissues, and also more radiation is absorbed by venous blood vessels than by surrounding tissues.

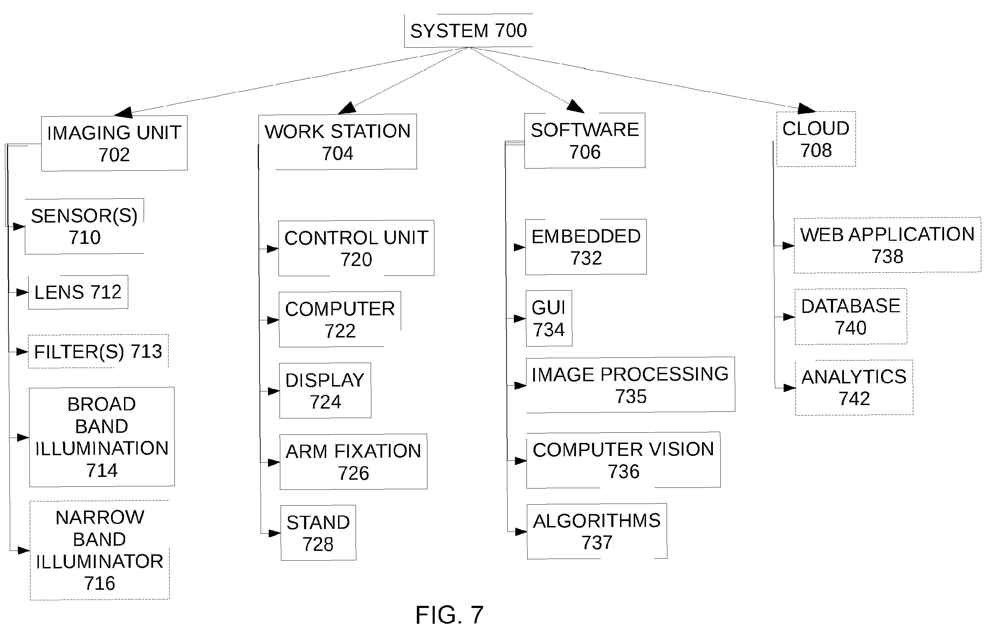

Reference is now made to Figure 7, which is a simplified block diagram of a system for measuring blood vessels according to an example embodiment of the disclosure.

Figure 7 shows a top-level block diagram of an example embodiment system 700. The system 700 may include an imaging/detection unit 702 and a software/computation unit 706.

The imaging/detection unit 702 optionally includes one or more sensor(s) 710, one or more lenses 712, one or more filter(s) 713, and one or more illuminator(s) 714 716.

In some embodiments the sensor(s) 710 may be CMOS sensor(s).

In some embodiments the sensor(s) 710 may be a multispectral and/or hyperspectral camera(s).

In some embodiments the sensor(s) 710 may be NIR sensor(s) or camera(s).

In some embodiments the lens 712 may optionally be a fixed focal length lens.

In some embodiments the lens 712 may optionally be a zoom lens.

In some embodiments the filter(s) 713 may optionally include bandpass or long-pass filter(s).

In some embodiments the illuminator(s) 714 716 may optionally include NIR LEDs, optionally in a spectral range of 700-1200 nm.

In some embodiments the illuminator(s) 714 716 may optionally include broad band NIR LEDs.

In some embodiments the illuminator(s) 714 716 may optionally include one or more laser sources, optionally in Near IR spectral range of 850nm and 910nm.

In some embodiments the illuminator(s) 714 716 may optionally include narrow band illumination, optionally in a spectral range of 900 nm

In some embodiments the illuminator(s) 714 716 may optionally include an array of illuminators.

In some embodiments the software/computation unit 706 optionally includes one or more of a GUI 734, an image processing unit 735, a computer vision unit 736, and a machine learning algorithm unit 737.

In some embodiments the algorithm unit 737 optionally includes one or more of: image processing algorithm(s), vein segmentation algorithm(s), collateral vein detection and/or segmentation algorithm(s), pulse wave detection algorithm(s), and classifier algorithm(s) - optionally machine learning algorithms.

The system 700 may include additional units, such as a work station 704, optional cloud infrastructure 708, etc.

In some embodiments the cloud infrastructure 708 optionally includes one or more of a web application 738, database(s) 740 (optionally including big data analytic capability), and analytic unit(s) 742.

In some embodiments, the work station 704 optionally includes one or more of:

a control unit 720, for controlling operation of the imaging/detection unit 702 and/or one or more of the components of the imaging/detection unit 702;
a computer 722;
a display 724;
an optional organ fixation surface or device 726, for optionally placing an organ at a specific location relative to the illumination 714 716 and/or the sensors 710; and
a stand 728, for placing components of the system at a specific location relative to a patient's organ.

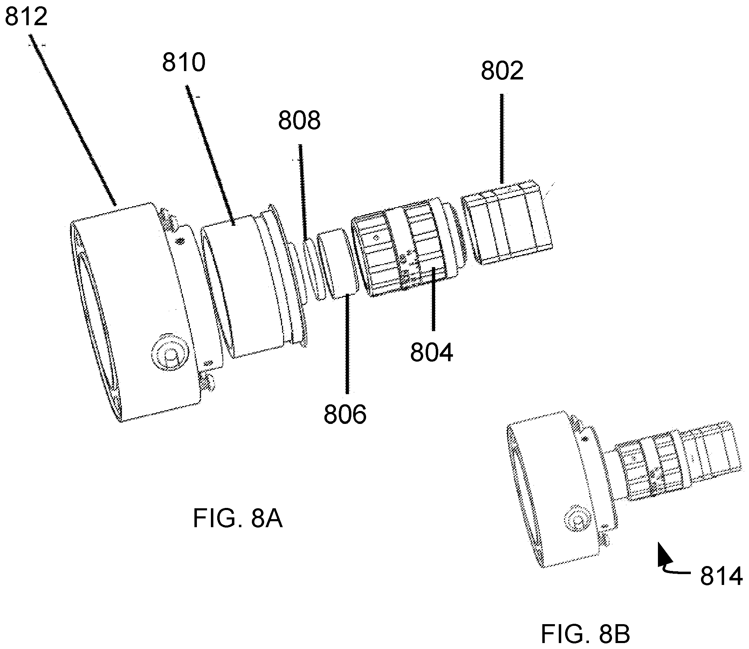

Reference is now made to Figures 8A and 8B, which are images of optical components in a system constructed according to an example embodiment of the disclosure.

Figures 8A and 8B show some of the example system's optical channel, which may include, as shown in Figure 8A:

a camera 802, optionally a hyper spectral sensor (camera);
a lens 802, optionally a fixed focal length lens;
an optional filter mount 806;
a filter 808, in some embodiments an optical long pass filter, in some embodiments a filter with a cut off wavelength of 670 nm; and
an illumination source 812.

In some embodiments the system includes an optional mechanical adaptor 810 to connect the illumination source 812 to the camera 802 body.

Figure 8B show an assembled unit 814 including the components of Figure 8A.

In some embodiments an example blood-vessel-status classifying algorithm may be divided to three blocks; image processing, feature extraction and statistical classifier.

The example algorithm TOP level flow may be similar to that shown in Figure 4A.

Image processing: The image processing block may include several steps:

- Image quality enhancement, such as contrast and illumination enhancement, sharpness, a combination of multiple polarization state images, multiple wavelength images (image of intensity ratios), multiple exposures, optionally High Dynamic Range (HDR), and contrast limited adaptive histogram equalization (CLAHE).

In some embodiments the intensity ratio images show a pixel-wise ratio between images that were captured with different wavelengths, as described the following equation:

$$R_{ij} = \frac{IM1_{ij}}{IM2_{ij}}$$

Where:
*Rij* is the pixel at location (i, j) in the ratio image, *IM1ij* is the pixel at location (i, j) in the first image, and *IM2ij* is the pixel at location (i, j) in the second image.

- Image segmentation - locating the vascular access (VA) construction, vessels' boundary and collateral vessels' structure.

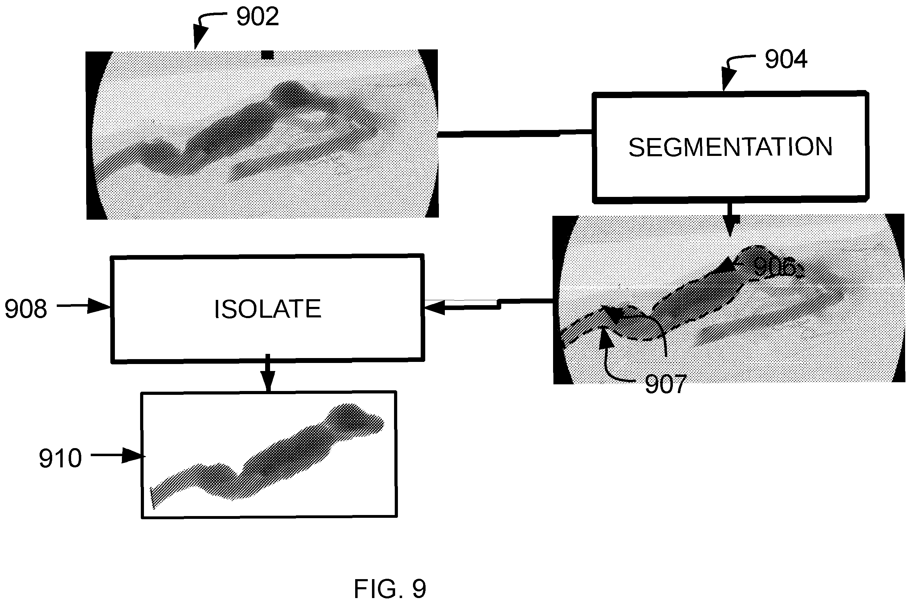

Reference is now made to Figure 9, which is a simplified flow chart illustration of a segmentation method according to an example embodiment of the disclosure.

Figure 9 shows an exemplary segmentation flow, including input of a first image 902, segmentation 904 of the first image 902 producing a second image 906 with optional segmentation lines 907, optionally isolating 908 an organ which appears in the second image 906, producing a third image 910 containing just an image of the isolated organ.

One or more of the following methods may be used: K-means algorithm, Histogram-based methods, Edge detection, Region-growing methods, Mumford and Shah Segmentation, CNN (convolutional neural networks), etc.

• Registration between an image or images from an earlier examination and from following examinations. The registration step optionally scales and/or aligns new image(s) to a reference image, optionally the image(s) from the earlier examination.

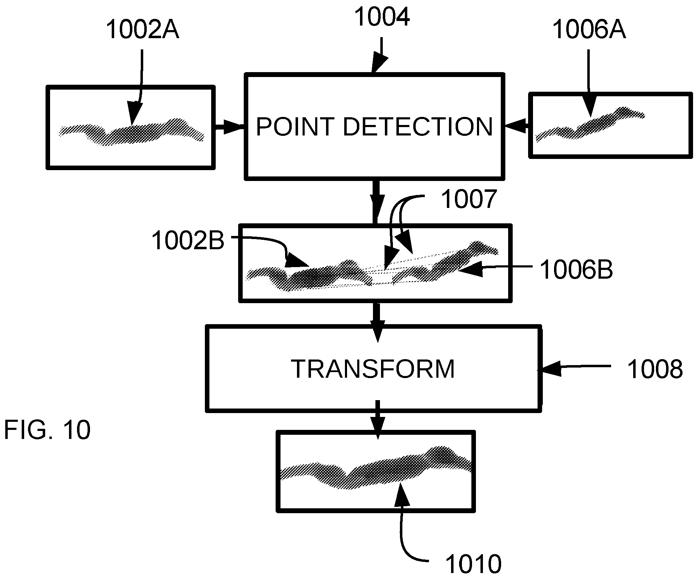

Reference is now made to Figure 10, which is a simplified flow chart illustration of a registration method according to an example embodiment of the disclosure.

Figure 10 shows a first image 1002A and a second image 1006A.

In some embodiments a point detection operation 1004 is optionally performed on the two images.

In some embodiments a point detection criterion is optionally one or more of: corner points, an intensity based criterion such as blob detection, SURF (speed up robust features), and so on.

In some embodiments, similarity of two points is measured by a feature metric difference between one or more feature metrics of each one of the two points.

The first image 1002A is marked by specific points detected in the first image 1002A, producing a first new image 1002B with specific points marked thereon. The second image 1006A is marked by specific points detected in the second image 1006A optionally according to same criteria used for detecting points in the first image 1002A, producing a second new image 1006B with specific points marked thereon.

Figure 10 shows some lines 1007 connecting corresponding specific points in the first new image 1002B and the second new image 1006B.

In some embodiments one or both of the first new image 1002B and the second new image 1006B are optionally transformed 1008, using the detection of corresponding marked points to perform the transformation, optionally producing a new combined image 1010. In some embodiments the transformation 1008 includes one or more of image standardization, image scaling, image rotation, and affine transform, performed on one or both of the first new image 1002B and the second new image 1006B.

In some embodiments the registration is performed to align and/or scale a first image, for example a current examination image, to a second image, for example a prior examination image. One or more of the following methods may be used for registration: SIFT (Scale Invariant Feature Transform), SURF (speeded up robust features) algorithm, optionally for interest-points detection, Automated Feature Detection and Matching, and Affine transform calculation.

• Feature extraction: A features extraction block may include several sub-blocks that analyze data and extract features from images.

In some embodiments the feature extraction optionally produces a feature vector.

In some embodiments the feature extraction is optionally performed after an image processing step which produces a standardized image.

The features vector is a mathematical representation used to characterize data such as an image. There are several ways to characterize the data, some of which are listed below:
Feature extraction from a pre-trained DNN (A. Krizhevsky, I. Sutskever, G. E. Hinton: ImageNet Classification with Deep Convolutional Neural Networks. NIPS 2012: 1106-1114).

One method includes passing an image through a neural network that was trained on a large image data set and use its descriptors layer.

Another way is to develop specific descriptors for each phenomenon.

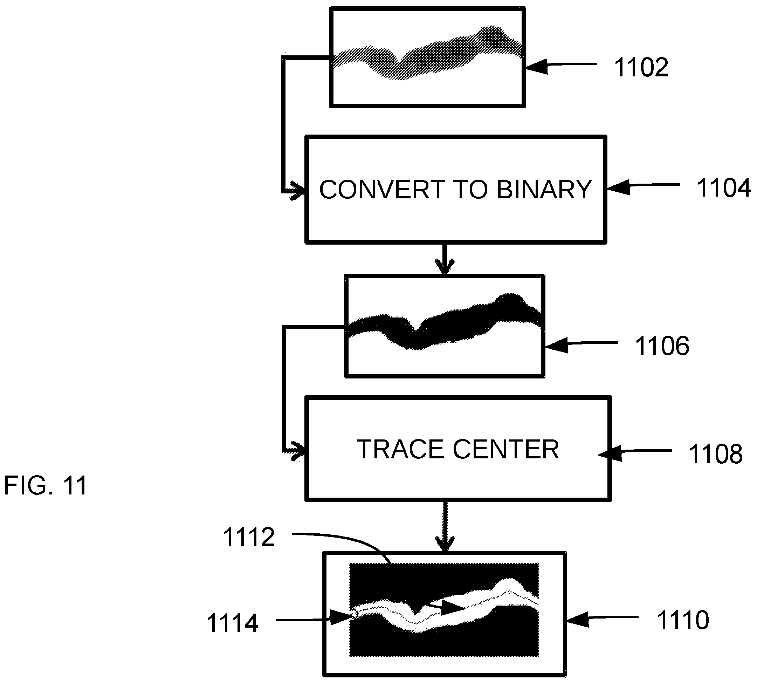

Blood vessel length and smallest diameter:
Reference is now made to Figure 11, which is a simplified flow chart illustration of a method according to an example embodiment of the disclosure.

Figure 11 illustrates a method producing a descriptor for a blood vessel's length and/or smallest diameter.

Figure 11 shows:

a first image 1102 as an input;
a conversion 1104 of the first image 1102 to a binary image 1106;
a location 1114 of a narrowest passage in the organ (blood vessel); and
a tracing 1108 of a center line of the organ (blood vessel) appearing in the binary image 1106, producing a third image 1110 with a center line 1112 of the organ (blood vessel) marked on the third image 1110.

A similar method can also optionally be used for producing a descriptor for "pulse wave velocity", "collateral vessels development", and "aneurysm and stenosis".

In the above method, "Distance transform" and "local maxima" methods may be used on a binary image for detecting the center line of the blood vessel and the diameter.

Other methods that can be useful are: Path finding algorithm -Dijkstra's algorithm, A* search algorithm.

Mixed arterial and venous oxygen concentrations in the VA:

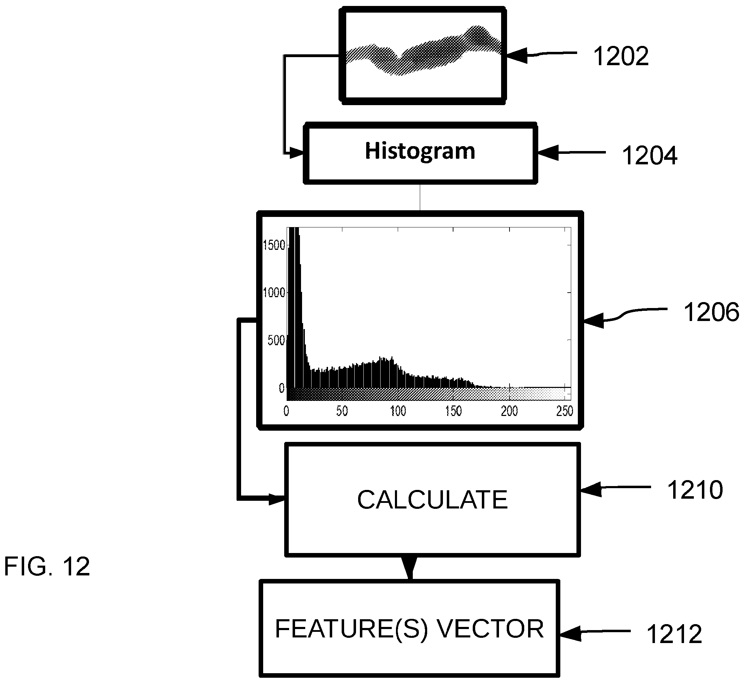

Reference is now made to Figure 12, which is a simplified flow chart illustration of a method according to an example embodiment of the disclosure.

Figure 12 illustrates a method producing a descriptor for arterial and/or venous oxygen concentrations in the VA.

Figure 12 shows:

a first image 1202 as an input;
a histogram unit 1204 for producing a histogram 1206 of the first image 1202; and
a calculation unit 1210 for producing a feature(s) vector 1212 associated with the first image 1202.

The absorption of deoxy Hb is higher than Oxy Hb at the range of 740nm to 760nm, so at this range, veins absorb the light radiation and arteries become relatively more transparent.

At the range of 850nm to 1000 nm the veins become relatively more transparent and arteries absorb more radiation.

The blood in the VA is a mixture of arterial and venous blood, especially when stenosis occurs, resulting in recirculation of blood.

As long as the VA functionality is good, a higher rate of arterial blood should flow though the VA, which is indicated by a darker gray level when illuminating at 850nm to 1000nm. By calculating the histogram for an intensity "standardized" image, such as the first image 1202 shown in Figure 12, and comparing the histogram to a histogram of a "reference" image, the system can create a features vector that describes a change in the blood mixture in the VA, or a rate of change in the blood mixture in the VA.

Pulse wave velocity

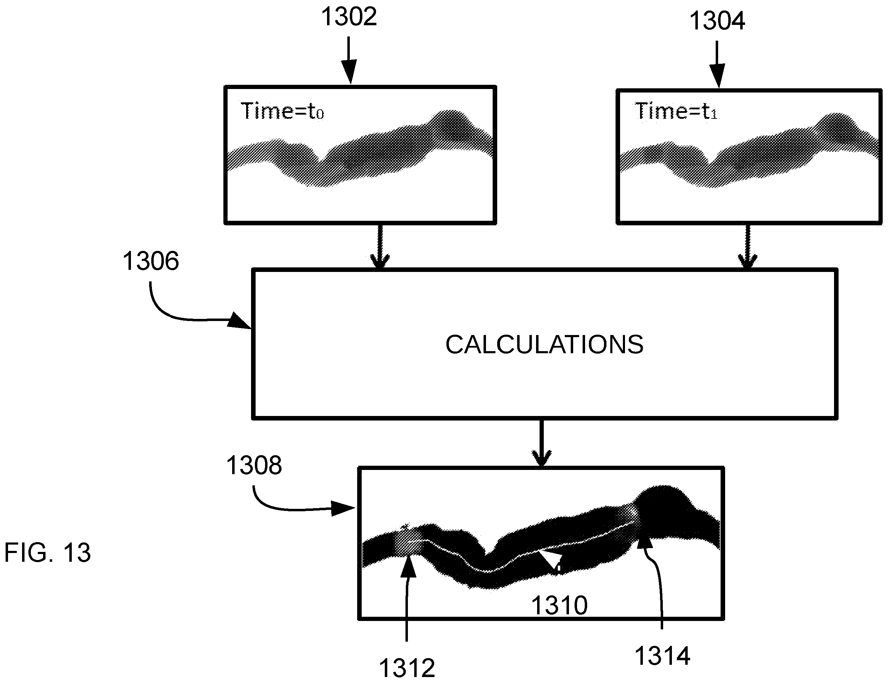

Reference is now made to Figure 13, which is a simplified flow chart illustration of a method according to an example embodiment of the disclosure.

Figure 13 illustrates a method for calculation of pulse wave velocity.

Figure 13 shows:

a first image 1302 obtained at a time $t_0$ as an input;
a second image 1304 obtained at a time $t_1$ as an input; and
a calculation unit 1306 for producing a third image 1308.

Figure 13 illustrates an exemplary method for features extraction of pulse wave velocity.

In some embodiments, two consecutive image frames, such as the images 1302 1304 of Figure 13, optionally each image frame after registration and/or segmentation (standardized images), are fused, producing a fused image such as the third image 1308.

In some embodiments the fused image is produced by subtraction of one of the images from the other.

In some embodiments the fused image is produced by adding one of the images to the other.

In some embodiments centers of mass of the two brightest spots 1312 1314 are calculated, and a length of a path 1312 between the centers of mass of the two brightest spots 1312 1314 along the path 1312 is measured.

In some embodiments the path 1312 is optionally a center line of the blood vessel.

Statistical classifier model

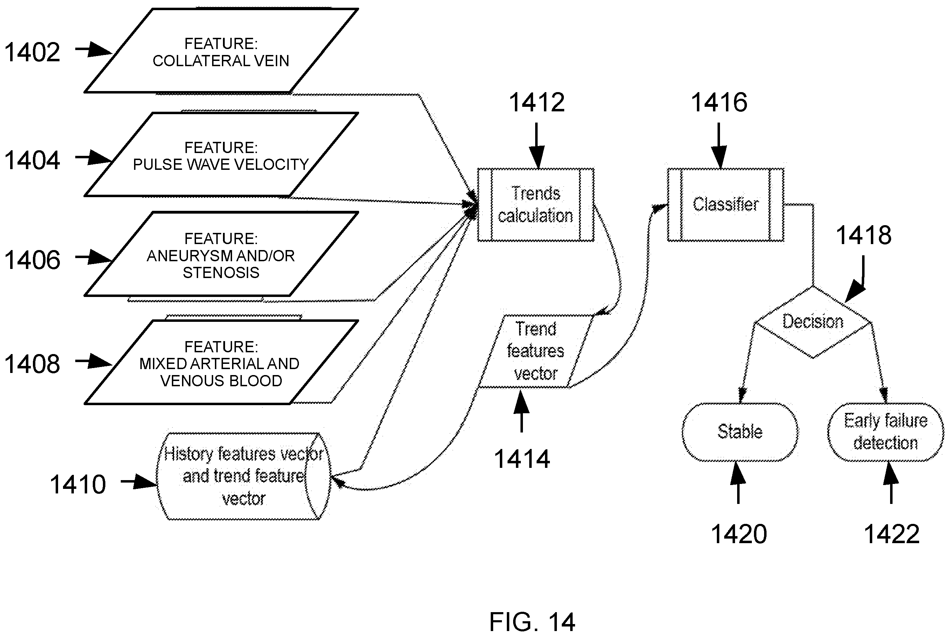

Reference is now made to Figure 14, which is a simplified flow chart illustration of a classifier method according to an example embodiment of the disclosure.

Figure 14 shows input of one or more feature descriptors, such as: a collateral vein descriptor 1402, a pulse wave velocity descriptor 1404, an aneurysm and/or stenosis descriptor 1406, and an arterial and venous blood mix descriptor 1408.

In some embodiments the inputs 1402 1404 1406 1408 are fed into a trend calculation unit 1412. In some embodiments the trend calculation unit 1412 optionally accepts input of a historical and/or trend descriptor 1410, optionally from a local or a remote database.

In some embodiments the trend calculation unit 1412 produces a trend features vector 1414.

In some embodiments the trend features vector 1414 is optionally stored in the (local or remote) database.

In some embodiments the trend features vector 1414 is input to a classifier 1416.

A result of the classifier 1416 is optionally input to a decision unit 1418, which produces a decision that the VA is determined to be "stable" 1420, or that a failure is detected 1422.

In some embodiments the detecting a failure may include estimating a high probability of imminent failure of the VA.

Classification to a "stable" or an "Early failure detection" can be done by a statistical classifier model, such as SVM, logistic regression, Neural network, etc.

In some embodiments extracted features 1402 1404 1406 1408 of every phenomenon are optionally collected to one "features" vector 1414.

In some embodiments the features vector 1414 is optionally stored in a data base.

In some embodiments the features vector 1414 and a "history features vectors" 1410 are optionally sent to a "Trends calculation" unit 1412.

In some embodiments the output of the "Trends calculation" unit 1412 is a "new trend features vector" 1414, which is optionally stored in the database and/or sent to a classifier unit 1416.

In some embodiments output from the classifier unit 1416 can be detected to be "Early failure detection" or "Stable".

In some embodiments, classification is made to a maturity level or rate of maturation after VA surgery can be done.

The rate of maturation of a fistula may be expressed as X% maturation after Y number of days.

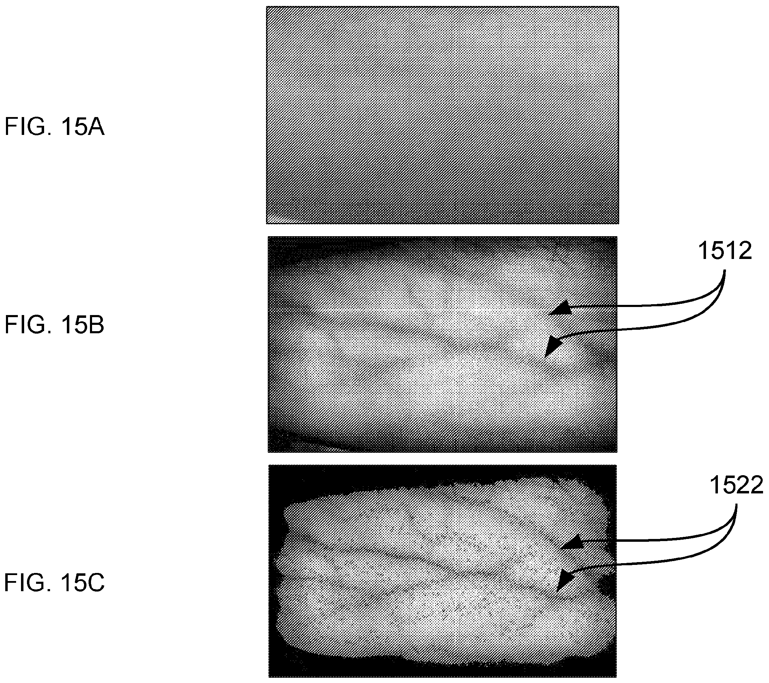

Reference is now made to Figures 15A-C, which show three different images of a same patient arm, according to an example embodiment of the disclosure.

Figure 15A shows an image of a patient's arm in human-visible wavelengths, taken at a distance of approximately 40 centimeters from the arm.

Figure 15B shows an image of a patient's arm in Near IR wavelengths. Figure 15B shows that using Near IR imaging improve visibility of blood vessels such as superficial veins 1512.

Figure 15C shows an image of a patient's arm, with points-of-interest 1522 which were automatically (by image analysis) generated at locations of the blood vessels.

When a nurse or physician examines a patient's blood vessels, they typically use a three-step procedure: look, listen and feel.

The claimed systems as described herein perform a look, listen and feel based on illuminating and imaging a patient's limb and analyzing the data collected from the imaging.

In some embodiments, methods as described herein performs a look, listen and feel based on illuminating and imaging a patient's limb and analyzing the data collected from the imaging.

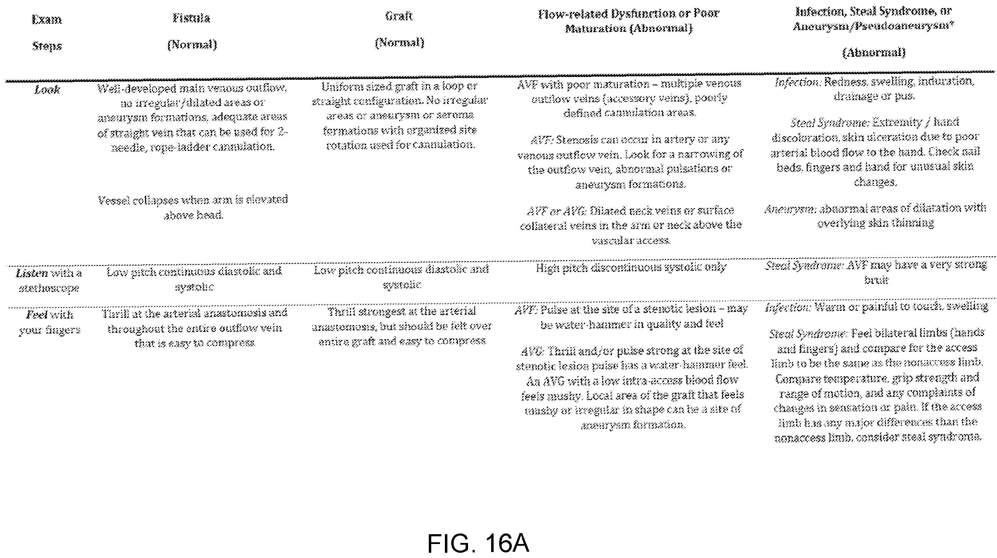

Reference is now made to Figure 16A, which is a table showing a procedure for a medical person to examine a patient with reference to vascular stenotic lesions or thrombosis.

Figure 16A is intended to show what a human is instructed to do. However, it is known that differences between humans is expected to affect such examinations.

It is noted that automatic examination is potentially able to provide better reproducibility for such examinations.

It is noted that automatic examination is potentially able to provide faster examinations with less involvement of medical staff.

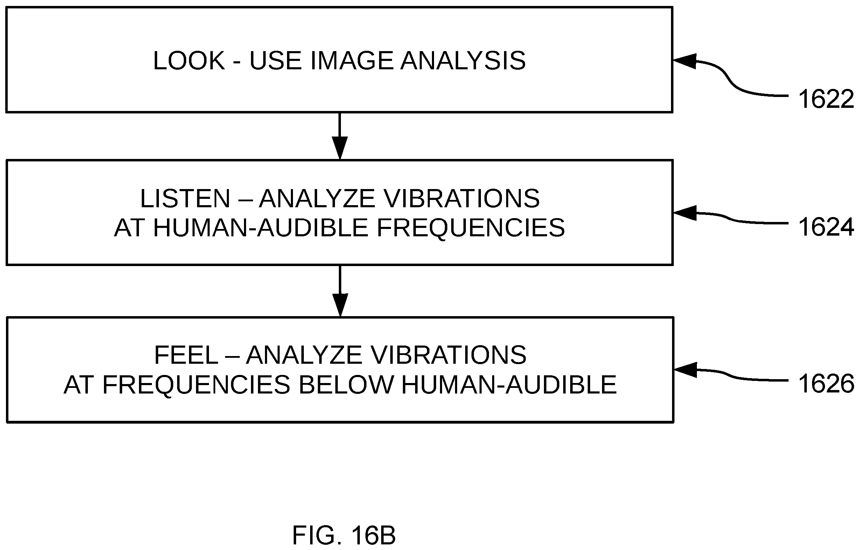

Reference is now made to Figure 16B, which is a simplified flow chart illustration of a method of examining a patient according to an example embodiment of the disclosure.

The method of Figure 16 includes:

    a device looking (1622) at a patient's body by capturing one or more images of the body, and using image analysis on the image(s);

the device listening (1624) to a patient's body by capturing vibrations of the body, and analyzing the vibrations at human-audible frequencies; and

the device feeling (1624) the patient's body by analyzing vibrations of the body, at frequencies below human-audible frequencies.

In some embodiments, the capturing one or more images of the body is optionally performed by capturing images at Near IR wavelengths.

In some embodiments, the capturing vibrations of the body is optionally performed by laser speckle imaging, as described elsewhere herein.

In some embodiments, the capturing vibrations of the body is optionally performed by a microphone touching the patient's body, and/or by a microphone attached to a stethoscope touching the patient's body.

It is noted that automatic examination, in some embodiments, is potentially able to provide such an examination without a human touching the patient, potentially usable in conditions where medical distancing is desired, such as, for example, when the patient may carry a contagious disease.

In some embodiments, the systems and methods described herein "look", that is, analyze images of blood vessels, "listen", that is, analyze vibration of the patient's body at human hearing frequencies, and "feel", that is, analyze vibration of the patient's body at low frequencies, reaching lower than typical audio frequencies.

In some embodiments, a no-contact surveillance tool is provided, to complement and/or replace physical examination of vascular access (VA). Such surveillance potentially enables early detection of stenosis, potentially earlier than human examination.

In some embodiments, the surveillance tool does not contact a patient's fistula, and/or a patient's limb, even while the limb is optionally positioned in a device which enables position the fistula in a field of view of the device.

In some embodiments, recording and monitoring parameters measure by the surveillance potentially enables the early detection and/or prediction of stenosis, potentially earlier than human examination.

In some embodiments, surveillance is enabled without human touch, for example at distances greater than 10, 20, 30, 40, 50 centimeters from a location of VA.

In some embodiments, the system and methods optionally enable acquiring all parameters typically acquired by a human physical examination by look, feel, and listen.

In some embodiments, it is easier to train persons to operate monitoring VA using embodiments as described herein than using the human senses.

Using embodiments as described herein, potentially add value by recording and using same-patient historical data and tracking changes.

Using embodiments as described herein potentially enable pre- and/or post- session examination in a clinic with no physical contact.

Using embodiments as described herein potentially support medical care under COVID-19.

Using embodiments as described herein potentially enable care in a home setup, possibly operated by a patient.

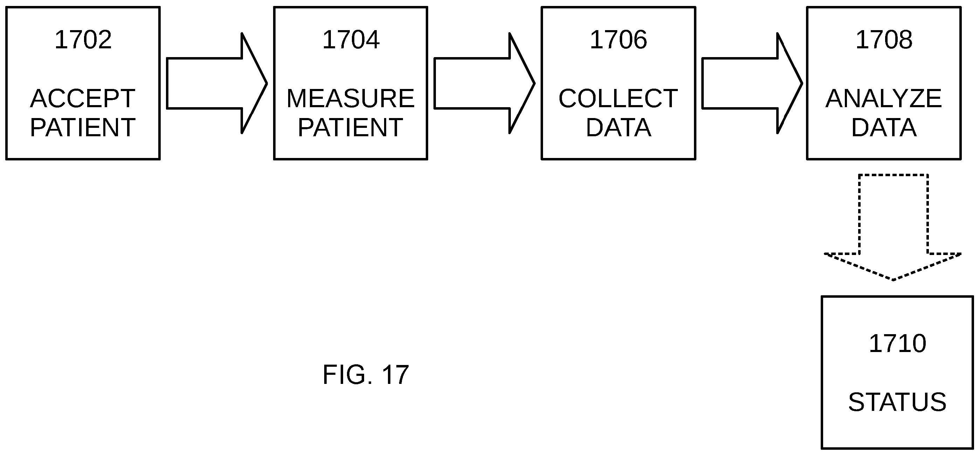

Reference is now made to Figure 17, which is a simplified block diagram illustration of a method for examining a patient according to an example embodiment of the disclosure.

Figure 17 shows a method including:

accepting a patient for examination (1702);
measuring the patient, using an embodiment of the disclosure (1704);
collecting data from sensors (1706);
analyzing the data (1708); and
optionally providing a decision (1710) regarding a status of the patient's fistula.

The status of the patient's fistula includes a determination of a medical condition and/or patency of the patient fistula. The medical condition is optionally determined to be healthy and/or functioning or having a probability of deterioration. In some embodiments a probability of deterioration above a certain threshold optionally produces a recommendation to send the patient to additional tests such as Doppler ultra-sonography or X-ray angiography.

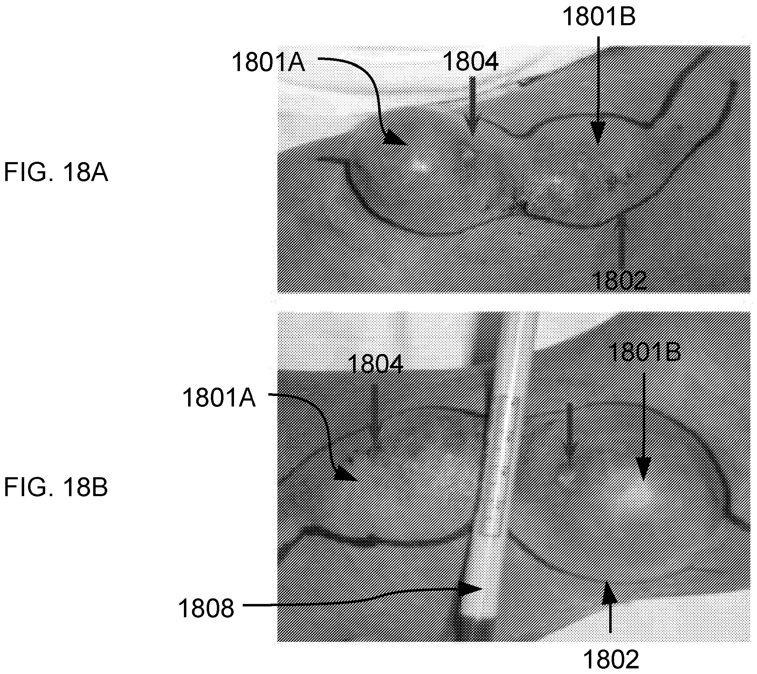

Reference is now made to Figures 18A and 18B, which are images of a fistula of a patient taken at two different times.

Figure 18A shows an ink marking 1802 of an outline of the fistula. Figure 18A also shows a physical feature 1804 visible on the skin of the patient.

Figure 18B is an image of the fistula taken at a different time. Figure 18B shows that the ink marking 1802 change shape due to a change in the shape and/or size of the fistula. Figure 18B also shows that the physical feature 1804 appears to have moved, relative to an outline of the fistula, or to the ink marking 1802.

Figures 18A and 18B are images of a 36-year-old man with a right brachiocephalic fistula created in 2004. The fistula has several aneurysms 1801A 1801B.

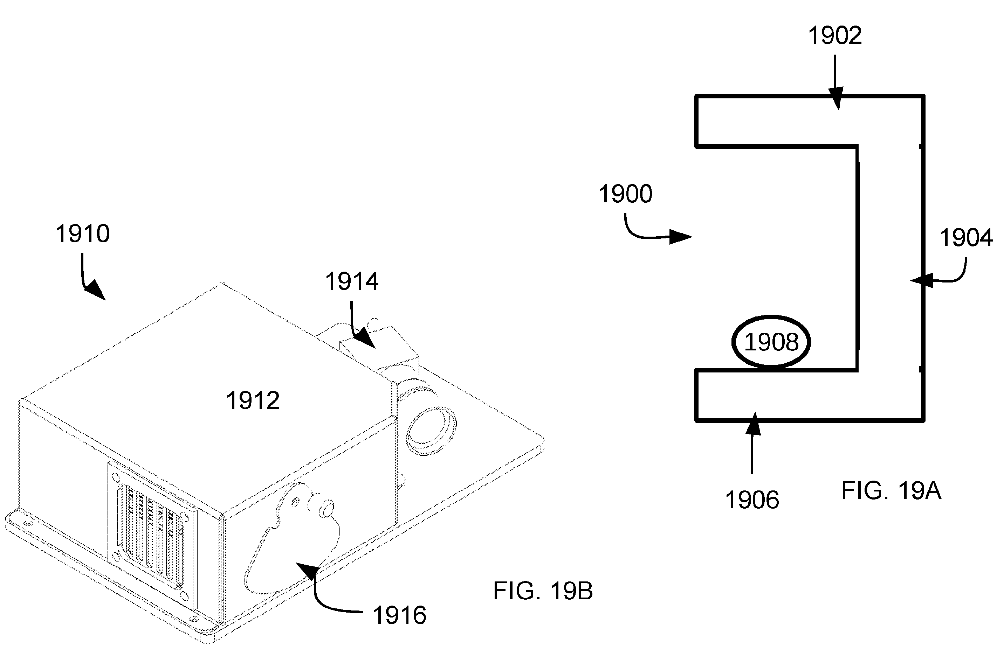

Reference is now made to Figures 19A and 19B, which are simplified drawings of a system for monitoring vascular access (VA) and/or fistulas according to two example embodiments of the disclosure.

Figure 19A shows a system 1900 including a head 1902 and a base 1906. The head 1902 optionally includes a light projector and an imaging system. In some embodiments, the base 1906 optionally includes a shape configured to support an arm or leg in a specific position relative to the head 1902. In some embodiments, the base 1906 optionally includes a strap configured to support an arm or leg in a specific position relative to the head 1902.

Figure 19A shows a system 1910 including a projector 1912 and an imager 1914. In some embodiments, the projector 1912 includes an optional cover 1916. In some embodiments, in especially in case when the projector includes a laser, the cover 1916 may be desired and/or required for safety.

In some embodiments, the imager 1914 is optionally capable of imaging frame at a rate above standard video rate, optionally at a rate of 60 Frames Per Seconds (FPS), above 60 FPS, above 100 FPS, above 150, 200, 300, 400, 500 and 600 FPS.

A high frame rate enables detecting vibrations of a patient's body at high frequencies, as is known in the art - Shannon's Law.

In some embodiments, a NIR fast Camera, optionally at frame rates of 150 FPS or greater, is optionally used.

In some embodiments, an off-the-shelf camera is optionally used, for example a FLIR FL3 U3 camera, capable of imaging at a frame rate of 150 FPS at a full frame size of 1.3 megapixels.

In some embodiments, the camera is used to capture a frame rate of more than 160 FPS, up to 600 FPS, 620 FPS and more.

In some embodiments, an off-the-shelf camera is optionally used, capable of imaging at a frame size in a range of 1.3-2 mega-pixels and more.

In some embodiments, an off-the-shelf camera is optionally used, capable of imaging at higher frame rates when imaging at a lower frame size. By way of some non-limiting examples, the camera optionally images at a size of 10x20 pixels, 10x10 pixels, and so on.

In some embodiments, the imager captures a small frame, less than maximum frame size and optionally down to the above-mentioned small frame sizes, of a specific location of interest on the patient's body at a location of the fistula or location of a VA point of interest.

In some embodiments, the projector optionally projects light onto the location of interest to enable a user to locate the patient's body correctly.

In some embodiments, the location of interest is a patient's fistula.

In some embodiments, more than one spot is illuminated simultaneously.

In some embodiments, one location of interest where a spot is illuminated is a patient's fistula, and another location of interest where a spot is illuminated is a location neighboring the patient's fistula, but not at the fistula.

In some embodiments, one location of interest where a spot is illuminated is a fistula aneurism, and another location of interest where a spot is illuminated is a location neighboring the fistula aneurism, but not at the fistula aneurism.

In some embodiments, the projector is a Digital Light Processing (DLP) projector.

In some embodiments, the projector is a laser projector.

In some embodiments, a location of interest, for example a fistula, or an aneurysm, or a bloated area of a body, is optionally identified by using structured lighting and image analysis, and the projector is controlled, optionally automatically controlled, to illuminate the location of interest. In some embodiments, the DLP and/or the laser projector are optionally controlled to illuminate the location of interest.

In some embodiments, a physician or nurse controls the illumination to the location of interest.

In some embodiments, a physician or nurse controls laser illumination to the location of interest.

In some embodiments, the projector is optionally capable of projecting light in multiple modes. The modes include two or more of:

projecting uniform (or approximately uniform) lighting on an area, or a limited spot, on a patient's body, potentially sufficient for imaging collateral veins; projecting structured lighting, optionally including stripes of specific widths, equal widths or unequal widths as programmed or other patterns; and projecting one or more spots of coherent laser light, potentially useful for measuring one or more of vibration, micro vibration, and pulses, for example by Laser Speckle Interferometry.

In some embodiments the projector is capable of switching between any one of three different lighting modes: uniform, structured and spot.

In some embodiments the projector is capable of providing a spot size in a range of diameters between 0.5 mm and 5 mm on a patient's limb. For example, a spot size of approximately 1 mm.

In some embodiments the projector includes one or more LEDs and/or laser light sources, optionally at Near IR wavelengths.

In some embodiments the projector is optionally a Digital Light Processing (DLP) projector.

In some embodiments the projector optionally includes nano-mirrors to shape light.

In some embodiments the projector optionally includes Micro-Electro-Mechanical System (MEMS) mirrors to shape light.

In some embodiments the projector optionally includes a Digital Mirror Driver (DMD).

In some embodiments, the projector and the camera are packaged in one package.

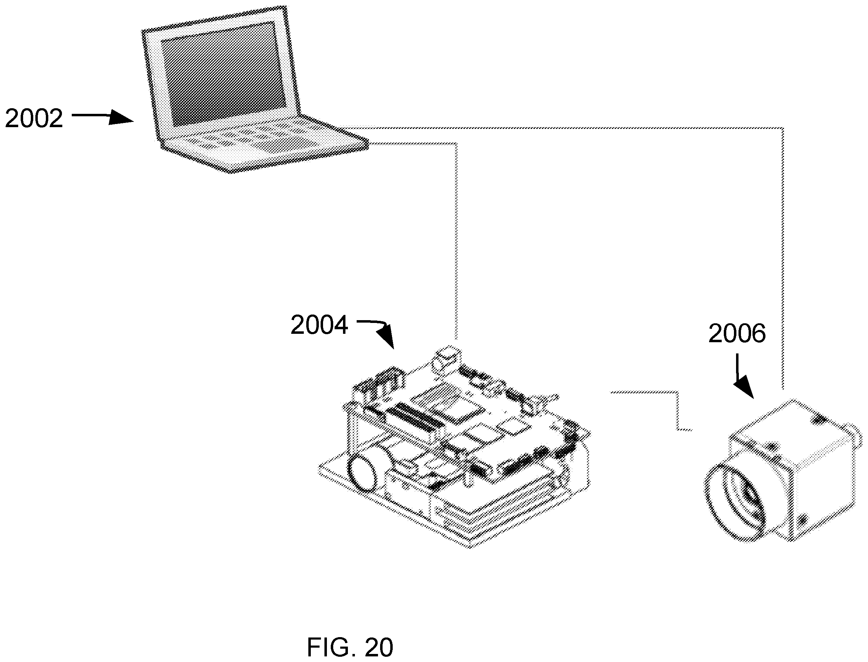

Reference is now made to Figure 20, which is an image of a system for monitoring vascular access (VA) and/or fistulas according to an example embodiment of the disclosure.

Figure 20 shows a system including a projector 2004, an imager 2006 and an optional processor 2002.

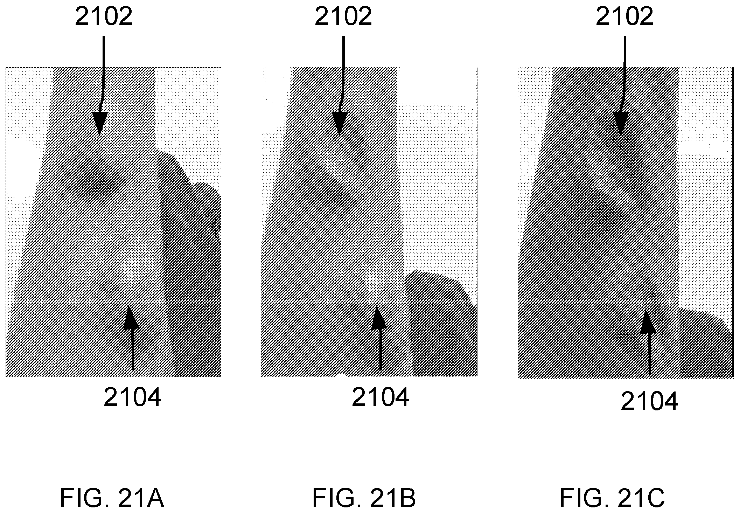

Reference is now made to Figures 21A-C, which show three different images of a same patient's arm.

The image of Figure 21A shows the patient's arm held below the patient's heart level. Two inflated needle insertion points 2102 2104 are shown on the patient's arm fistula.

The image of Figure 21B shows the patient's arm held above the patient's heart level, the image captured just after the patient raised the arm to the elevated position. A first 2102 one of the insertion points is shown deflated, and a second 2104 of the insertion points is still inflated.

The image of Figure 21C shows the patient's arm held elevated, the image captured a little later than the image of Figure 21B. Both of the needle insertion points 2102 2104 are shown deflated and collapsed.

Examination with elevation test

In some embodiments, systems and methods described herein are optionally used to measure and quantify in an elevation test, that is, the measurement and quantification are performed once or more with a body or limb held below the heart level, and once or more with the body or limb held at an elevated position above heart level.

As Figures 21A-C show, in some instances a fistula may not drain when held at one position, and drain when held at one or more other positions. In some instances a fistula may not drain when held at one position, and also not drain when held at one or more other positions.

Differences between the positions correlate with medical condition of the fistula such as a ratio between inflow and outflow rates and/or pressure).

The rate and manner at which the fistula drains, as well as the difference between draining pattern while at different elevations correlates with medical condition of the fistula. In some embodiment, drainage rate and/or pattern are measured, optionally by generating a 3D shape and/or one or more 3D curves depicting the outer shape of the fistula and/or tracing changes between curves along the set.

In some embodiments, the drainage rate and pattern are optionally estimated by evaluating the volume encapsulated by the 3D shape and/or by one or more curve(s) and tracing the change in total volume over time, potentially providing a level and rate of draining.

In some embodiment a spatial curvature of a curve(s) can be estimated, and drainage pattern can be characterized by analyzing the changes in curvature over time.

In some embodiment the smoothness of curvature of each curve and changes in curvature smoothness over time during drainage can be used for estimation of a drainage pattern.

In some embodiment correlation between any flow related parameter estimated from analysis of measured vibrations and any parameter estimated from the drainage pattern or rate based on 3D shape or curve shape(s) is optionally used for estimating fistula health.

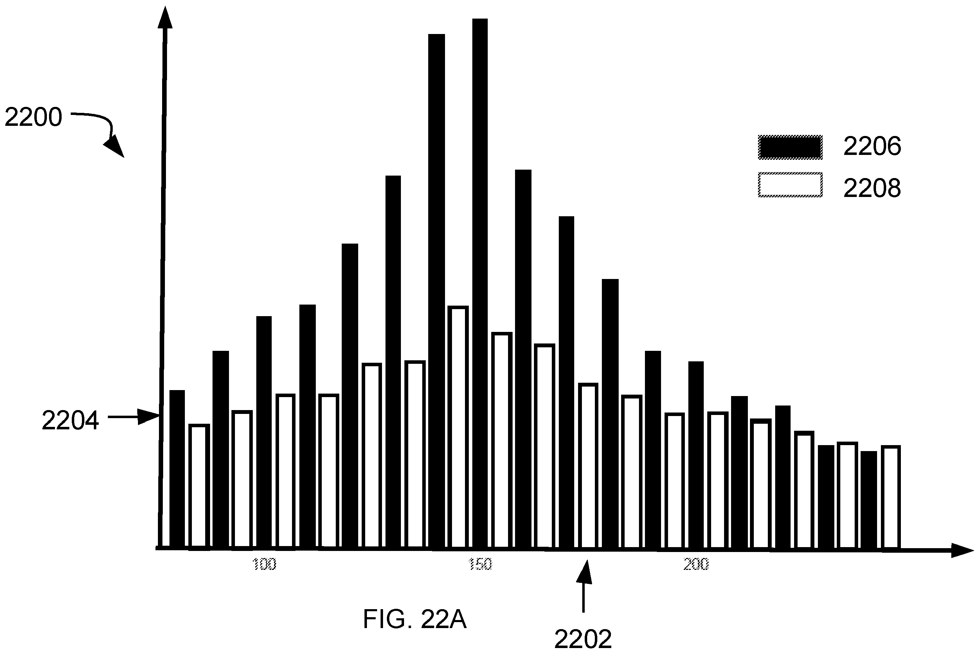

Reference is now made to Figure 22A, which is a graph showing power spectrum of vibrations measured by analysis of images produced by laser speckle imaging.

Figure 22A shows a graph 220, with a X-axis 2202 showing frequency ranges or bins, and a Y-axis 2204 showing relative power spectrum in the units in which it was measured.

Two groups of patients were sampled for producing this graph. A first group 2206 having a blood flow velocity (FV) greater than 500 mL/minute, and a second group 2208 having FV less than 500 mL/minute.

The graph 2200 shows us that the maximum in the power spectrum is located approximately at approximately 140 Hz for both groups. This leads us to suspect that listening to the pitch of the blood flow in both groups might not be a good method to differentiate among them. However, analyzing the power spectrum of both groups shows differences:

The first group 2206 appears to have a higher amplitude at the maximum than the second group 2208; The second group 2208 appears to have a flatter, or broader, curve than the first group 2206.

The vibrations analyzed in the power spectrum, as shown in Figure 22A, are caused by blood flow and/or turbulence through a blood vessel.

Flow and turbulence change over time and are affected by local physical conditions in and around the vessels through which the flow occurs. The physical conditions potentially include a pressure gradient, vessel diameter, vessel wall compliance, vessel inner surface characteristics, and so on.

The power spectrum of blood flow measured at VA / fistula locations is potentially related to physical and/or clinical flow conditions at these locations. Changes in the features of such power spectra over time potentially correlate to degradation in fistula health. Analyzing the changes in the power spectrum obtained from the VA / fistula location are potentially early stage predictive of fistula deterioration.

It is noted with reference to early stage predictions described herein, that such predictions potentially enable performing percutaneous transluminal angioplasty (PTA) earlier than would be performed based on the existing state of medical examination.

In some embodiments, the power spectrum is measured by measuring an intensity of light reflected off a patient's body. The intensity is expected to change at a frequency related to frequency of vibration of the body.

In some embodiments, the power spectrum is measured by measuring an intensity of light reflected off an illumination spot on the patient's body. In such embodiments the vibration is practically measured specifically at the illuminated spot.

In some embodiments, the power spectrum is measured by measuring differences between successive images of the body, for example small shifts of a pattern on the body. The pattern may be a mole on the skin, structured lighting, movement of a spot of light, movement of laser speckles, and similar movements.

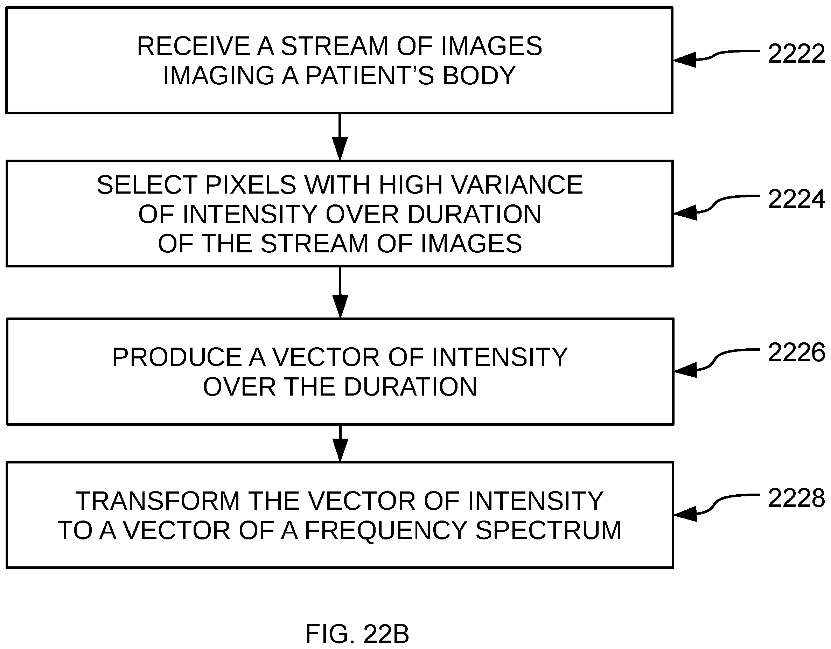

Reference is now made to Figure 22B, which is a simplified flow chart illustration of a method for transforming data from a stream of images to a frequency spectrum according to an example embodiment of the disclosure.

The method of figure 22B includes:

receiving a stream of images imaging a patient's body (2222);
optionally selecting one or more pixels with high variance of intensity over duration of the stream of images (2224);
producing a vector of intensity over the duration (2226);
transforming the vector of intensity to a vector of a frequency spectrum (2228).

In some embodiments, the transforming is performed by a Fast Fourier Transform.

In some embodiments, before analyzing the power spectrum, the power spectrum is optionally normalized. By way of some non-limiting examples, a normalization factor is optionally calculated from: total spectrum energy, peak value, peak to baseline ratio, energy in a specific band width, and so on.

In some embodiments, a reference spectrum measured at a remote location (far from the fistula, on the other hand for example), is used as a reference. Both spectrums may or may not be normalized and the measured spectrum replaces by a difference between the spectra at the different locations.

In some embodiments, skewness or kurtosis of the measured power spectrum or the difference power spectrum are optionally used for estimating flow.

In some embodiments, a measured power spectrum is first fitted to a model, in some embodiments assuming one or more hidden model mixtures, by way of a non-limiting example a Poisson-Gaussian mixture, and model parameters are used as correlators to flow.

In some embodiments, energy in a specific frequency range is used for estimating flow.

Performing Look, Listen and Feel

We additionally describe some aspects of the invention in terms of a procedure of "Look, Listen and Feel" which is used by medical staff.

In some embodiments, a "Look, Listen and Feel" procedure is performed by embodiments of the system described herein.

In some embodiments, systems as described herein perform a look, listen and feel based on illuminating and imaging a patient's limb and analyzing the data collected from the imaging.

In some embodiments, methods as described herein performs a look, listen and feel based on illuminating and imaging a patient's limb and analyzing the data collected from the imaging.

A fistula bruit, also called a vascular murmur, is an indicator of how dialysis access is functioning.

An arteriovenous fistula is one access type that is created by connecting an artery to a vein under the skin, usually in the upper or lower arm or leg. (i) The high blood flow from the artery through the vein allows the fistula to grow larger and stronger. A healthy AV fistula has a bruit (a rumbling sound that a human can hear), a thrill (a rumbling sensation that a human can feel), and good blood flow rate.

In some embodiments, the "Look" aspect is optionally performed by imaging a body or limb and analyzing an image or images to quantify blood vessel structure and/or fistula structure.

In some embodiments, the "Look" aspect is optionally performed by imaging a body or limb using structured light, and producing a 3D shape of a fistula.

In some embodiments, the "Listen" aspect is optionally performed by measuring vibrations of a body or limb and analyzing the vibrations to quantify parameter values relating to a medical condition of a fistula. In some embodiments, the "Listen" aspect includes analyzing vibrations in a frequency range within the human audible range.

In some embodiments, the "Feel" aspect is optionally performed by measuring vibrations of a body or limb and analyzing the vibrations to quantify parameter values relating to a medical condition of a fistula. In some embodiments, the "Feel" aspect includes analyzing vibrations optionally in a frequency range extending even beyond and/or below the human audible range.

In some embodiments, analyzing vibrations is optionally performed in a frequency range of less than 1,000 Hz. In some embodiments, analyzing vibrations is optionally performed in a frequency range of less than a typical human speech, for example less than 4,000 Hz.

By way of some non-limiting examples, the "Feel" aspect includes one or more of:
Measuring human pulse, which is typically in a range of 40 beats per minute and above. Such measurement needs analyzing vibrations at a frequency of 1 Hertz and even somewhat less. When such analyzing is performed by analyzing image frames of a video sequence, it is sufficient to analyze image frames at approximately double the rate of the frequency being measured, that is, for example, approximately 2 frames per second or above.

Measuring thrill, which is typically in a range of 50-250 Hertz or 50-750 Hertz. When such analyzing is performed by analyzing image frames of a video sequence, it is sufficient to analyze image frames at approximately double the rate of the frequency being measured, that is, for example, approximately 100 frames per second or above.

Analyzing and quantifying a power spectrum of the vibrations, for example as described above with reference to Figures 22A and 22B.

In some embodiments, the "Look, Listen and Feel" is performed without physically touching the patient, by image analysis and/or by using a specific mode of lighting.

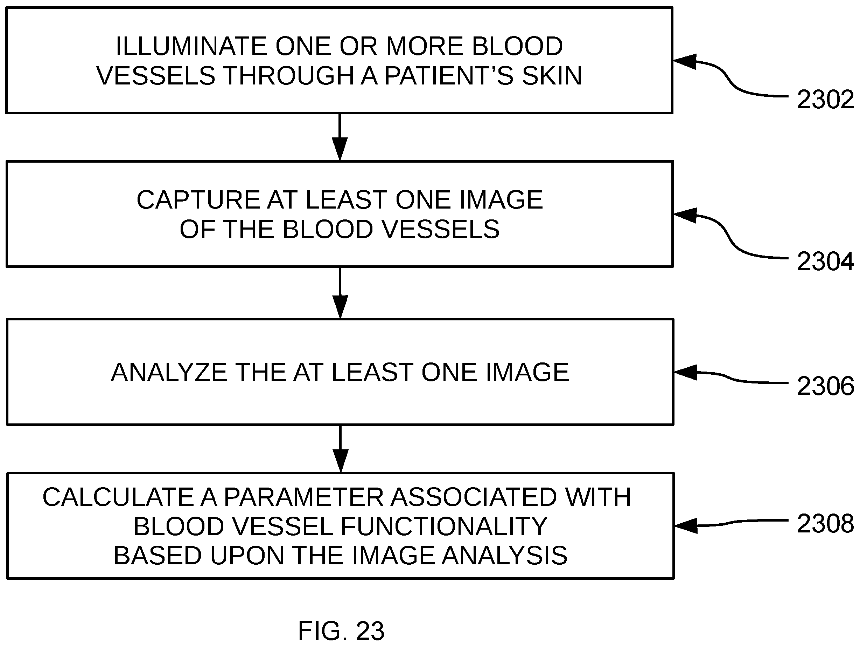

Reference is now made to Figure 23, which is a simplified flow chart illustration of a method for monitoring blood vessel functionality according to an example embodiment of the disclosure.

The method of Figure 23 includes:

illuminating one or more blood vessels through a patient's skin (2302);
capturing at least one image of the blood vessels (2304);
analyzing the at least one image (2306); and
calculating a parameter associated with blood vessel functionality based upon the image analysis (2308).

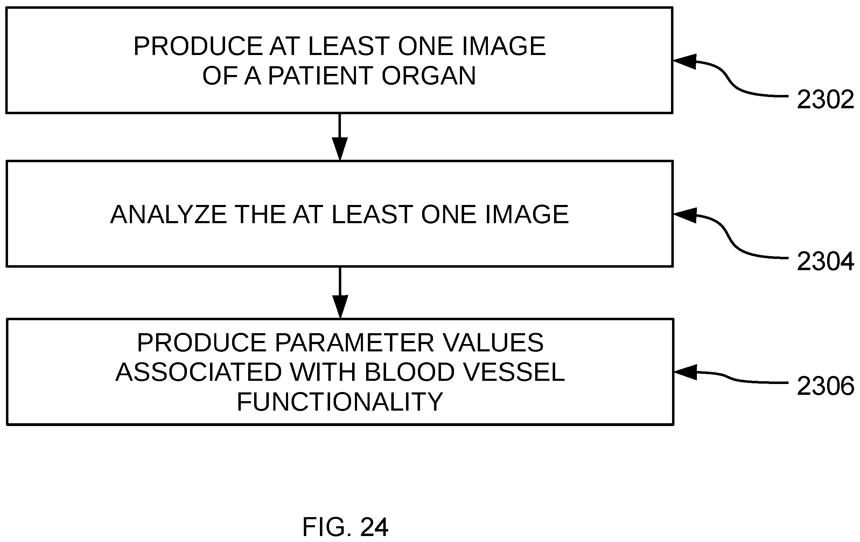

Reference is now made to Figure 24, which is a simplified flow chart illustration of a method for replacing a physical examination performed by medical staff for monitoring blood vessel functionality according to an example embodiment of the disclosure.

The method of Figure 24 includes:

producing at least one image of a patient organ (2402);
analyzing the at least one image (2404); and
producing parameter values associated with blood vessel functionality (2406).

It is expected that during the life of a patent maturing from this application many relevant image processing algorithms will be developed and the scope of the term image processing is intended to include all such new technologies *a priori*.

As used herein with reference to quantity or value, the term "approximately" means "within ± 15 % of".

The terms "comprising", "including", "having" and their conjugates mean "including but not limited to".

The term "consisting of" is intended to mean "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a unit" or "at least one unit" may include a plurality of units, including combinations thereof.

The words "example" and "exemplary" are used herein to mean "serving as an example, instance or illustration". Any embodiment described as an "example or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

**Claims**

1. A system **(200)** for monitoring vascular access in patients undergoing hemodialysis treatments by an automatic look listen feel examination, the system comprising:

    a camera **(204)** configured to image a patient's body **(212)** to obtain blood vessel geometry;
    an illumination source **(202)**; and
    a processor **(208)** configured to receive an image from the camera **(204)**,
    the system **(200)** configured to:

        obtain a shape of a body organ at a location of a vascular access (VA) of the patient's body **(212)** using image analysis; and
        analyze vibrations of the patient's body **(212)** at a location of the patient's body which includes blood vessels using image analysis,
        wherein
        the system is programmed to automatically perform a look, listen, and feel examination of the patient's body by:

            implementing the look by image analysis of images obtained by the imaging;
            implementing the listen by the analyzing vibrations, at human audible frequencies, by image analysis of the images obtained by the imaging; and
            implementing the feel by the analyzing vibrations at frequencies extending to below human audible frequencies, by image analysis of the images obtained by the imaging.

2. A system **(200)** according to claim 1 comprising a classifier configured to combine two or more features extracted from the look, the listen and the feel, to classify a condition of the VA.

3. A system (200) according to any one of claims 1-2 wherein the obtaining a shape of a body organ comprises calculating a three-dimensional (3D) shape of the patient's fistula.

4. A system (200) according to claim 2 configured to record the features measured along a surveillance period and predicting likelihood of stenosis based on changes along the surveillance period.

5. A system (200) according to any one of claims 1-4 wherein said illumination source (202) is configured to switch projecting light between at least two out of three modes of projecting light: uniform illumination, structured light illumination and coherent laser light for spot illumination.

6. A system (200) according to any one of claims 1-5 comprising a camera capable of capturing images at a frame rate greater than 150 Frames Per Second (FPS).

7. A system (200) according to any one of claims 1-5 wherein the illumination source (202) comprises a Digital Light Processing (DLP) projector.

8. A system (200) according to any one of claims 1-7 configured to calculate a rate of evacuation of a patient's fistula based on changes in a 3D shape of the patient's fistula.

9. A system (200) according to claim 8, wherein said calculation is performed during an elevation test.

10. A system (200) according to any one of claims 1-9 configured to calculate one or more parameters indicative of development of one or more collateral vessels based upon the image analysis.

11. A system (200) according to any one of claims 1-10 wherein the system is configured to provide feedback to support a remote physical examination.

12. A system (200) according to any one of claims 1-11 configured to calculate one or more parameters based upon the image analysis and to calculate an estimation of a maturity of a VA based on a rate of change of the one or more parameters.

13. A system (200) according to any one of claims 1-12 configured to calculate one or more parameters based upon passing an image through a trained neural network and using a descriptors layer of the trained neural network.

**Patentansprüche**

1. System (200) zur Überwachung eines vaskulären Zugangs bei Patienten, die einer Hämodialysebehandlung unterzogen werden, durch eine automatische visuelle, akustische und taktile Untersuchung, wobei das System umfasst:

eine Kamera (204), die dazu ausgestaltet ist, einen Körper (212) eines Patienten abzubilden, um eine Blutgefäßgeometrie zu erhalten; eine Beleuchtungsquelle (202); und einen Prozessor (208), der dazu ausgestaltet ist, ein Bild von der Kamera (204) zu erhalten, wobei das System (200) ausgestaltet ist zum:

Erhalten einer Form eines Körperorgans an einer Stelle eines vaskulären Zugangs (VA) des Körpers (212) des Patienten unter Verwendung von Bildanalyse; und Analysieren von Schwingungen des Körpers (212) des Patienten an einer Stelle des Körpers des Patienten, die Blutgefäße umfasst, unter Verwendung von Bildanalyse,

wobei das System programmiert ist, um eine visuelle, akustische und taktile Untersuchung des Körpers des Patienten automatisch durchzuführen durch:

Implementieren der visuellen Untersuchung durch Bildanalyse von Bildern, die durch das Abbilden erhalten werden; Implementieren der akustischen Untersuchung durch Analysieren von Schwingungen bei für Menschen hörbaren Frequenzen durch Bildanalyse der durch das Abbilden erhaltenen Bilder; und Implementieren der taktilen Untersuchung durch Analysieren von Schwingungen bei Frequenzen, die sich unter die für Menschen hörbaren Frequenzen erstrecken, durch Bildanalyse der durch das Abbilden erhaltenen Bilder.

2. System (200) nach Anspruch 1, das eine Klassifikator, der dazu ausgestaltet ist, zwei oder mehr Merkmale zu kombinieren, die von der visuellen, akustischen und taktilen Untersuchung extrahiert werden, um einen Zustand des VA zu klassifizieren.

3. System (200) nach einem der Ansprüche 1 bis 2, wobei das Erhalten einer Form eines Körperorgans das Berechnen einer dreidimensionalen (3D) Form der Fistel des Patienten umfasst.

4. System (200) nach Anspruch 2, das dazu ausgestaltet ist, die Merkmale, die über eine Überwachungsperiode gemessen werden, aufzuzeichnen und eine Wahrscheinlichkeit einer Stenose auf der Grundlage von Änderungen über die Überwachungsperiode vorherzusagen.

5. System (200) nach einem der Ansprüche 1 bis 4, wobei die Beleuchtungsquelle (202) dazu ausgestaltet ist, projiziertes Licht zwischen mindestens zwei von drei Modi des Projizierens des Lichts zu schalten: gleichförmige Beleuchtung, Beleuchtung mit strukturiertem Licht und kohärentes Laserlicht zur Punktbeleuchtung.

6. System (200) nach einem der Ansprüche 1 bis 5, umfassend eine Kamera, die in der Lage ist, Bilder bei einer Bildfrequenz von größer als 150 Bildern pro Sekunde (Frames Per Second - FPS) aufzunehmen.

7. System (200) nach einem der Ansprüche 1 bis 5, wobei die Beleuchtungsquelle (200) einen Digital Light Processing (DLP) Projektor umfasst.

8. System (200) nach einem der Ansprüche 1 bis 7, das dazu ausgestaltet ist, eine Abflussrate einer Fistel eines Patienten auf der Grundlage von Änderungen bei einer 3D-Form der Fistel des Patienten zu berechnen.

9. System (200) nach Anspruch 8, wobei die Berechnung während eines Elevationstests durchgeführt wird.

10. System (200) nach einem der Ansprüche 1 bis 9, das dazu ausgestaltet ist, einen oder mehrere Parameter, die eine Entwicklung eines oder mehrerer Kollateralgefäße angeben, auf der Grundlage der Bildanalyse zu berechnen.

11. System (200) nach einem der Ansprüche 1 bis 10, wobei das System dazu ausgestaltet ist, eine Rückmeldung zur Unterstützung einer physikalischen Fernuntersuchung bereitzustellen.

12. System (200) nach einem der Ansprüche 1 bis 11, das dazu ausgestaltet ist, einen oder mehrere Parameter auf der Grundlage der Bildanalyse zu berechnen und eine Schätzung einer Reife eines VA auf der Grundlage einer Änderungsrate des einen oder der mehreren Parameter zu berechnen.

13. System (200) nach einem der Ansprüche 1 bis 12, das dazu ausgestaltet ist, einen oder mehrere Parameter auf der Grundlage des Durchlaufs eines Bildes durch ein trainiertes neuronales Netzwerk und unter Verwendung einer Deskriptorenschicht des trainierten neuronalen Netzwerks zu berechnen.

**Revendications**

1. Système (200) destiné à surveiller l'accès vasculaire chez des patients soumis à des traitements d'hémodialyse par un examen automatique consistant à regarder, écouter et palper, le système comprenant :

une caméra (204) configurée pour filmer le corps d'un patient (212) afin d'obtenir la géométrie des vaisseaux sanguins ;
une source d'éclairage (202) ; et
un processeur (208) configuré pour recevoir une image provenant de la caméra (204),
le système (200) étant configuré pour :

obtenir la forme d'un organe corporel à l'emplacement d'un accès vasculaire (VA) du corps du patient (212) à l'aide d'une analyse d'image ; et
analyser les vibrations du corps du patient (212) à un emplacement du corps du patient qui comprend des vaisseaux sanguins à l'aide d'une analyse d'image,
dans lequel
le système est programmé pour effectuer automatiquement un examen visuel, auditif et tactile du corps du patient en :

mettant en œuvre l'examen visuel par analyse d'images obtenues par imagerie ;
mettant en œuvre l'écoute par l'analyse des vibrations, à des fréquences audibles par l'oreille humaine, par analyse d'images obtenues par imagerie ; et
mettant en œuvre le toucher par l'analyse des vibrations à des fréquences s'étendant en dessous des fréquences audibles par l'oreille humaine, par analyse d'images obtenues par imagerie.

2. Système (200) selon la revendication 1, comprenant un classificateur configuré pour combiner deux ou plusieurs caractéristiques extraites de l'examen visuel, auditif et tactile, afin de classer un état de l'AV.

3. Système (200) selon l'une quelconque des revendications 1 à 2, dans lequel l'obtention d'une forme d'un organe corporel comprend le calcul d'une forme tridimensionnelle (3D) de la fistule du patient.

4. Système (200) selon la revendication 2, configuré pour enregistrer les caractéristiques mesurées au cours d'une période de surveillance et prédire la probabilité de sténose sur la base des changements observés au cours de la période de surveillance.

5. Système (200) selon l'une quelconque des revendications 1 à 4, dans lequel ladite source d'éclairage (202) est configurée pour commuter la lumière projetée entre au moins deux des trois modes de projection de lumière d' : éclairage uniforme, éclairage par lumière structurée et lumière laser cohérente pour l'éclairage ponctuel.

6. Système (200) selon l'une quelconque des revendications 1 à 5, comprenant une caméra capable de capturer des images à une fréquence d'images supérieure à 150 images par seconde (FPS).

7. Système (200) selon l'une quelconque des revendications 1 à 5, dans lequel la source d'éclairage (202) comprend un projecteur à traitement numérique de la lumière (DLP).

8. Système (200) selon l'une quelconque des revendications 1 à 7, configuré pour calculer un taux d'évacuation de la fistule d'un patient sur la base des changements dans la forme 3D de la fistule du patient.

9. Système (200) selon la revendication 8, dans lequel ledit calcul est effectué pendant un test d'élévation.

10. Système (200) selon l'une quelconque des revendications 1 à 9, configuré pour calculer un ou plusieurs paramètres indicatifs du développement d'un ou plusieurs vaisseaux collatéraux sur la base de l'analyse d'image.

11. Système (200) selon l'une quelconque des revendications 1 à 10, dans lequel le système est configuré pour fournir un retour d'information afin de faciliter un examen physique à distance.

12. Système (200) selon l'une quelconque des revendications 1 à 11, configuré pour calculer un ou plusieurs paramètres sur la base de l'analyse d'image et pour calculer une estimation de la maturité d'un VA sur la base d'un taux de variation du ou des paramètres.

13. Système (200) selon l'une quelconque des revendications 1 à 12, configuré pour calculer un ou plusieurs paramètres sur la base du passage d'une image à travers un réseau neuronal entraîné et en utilisant une couche de descripteurs du réseau neuronal entraîné.

FIG. 1

206

208

Control unit

Processor

210

Display

204

Illumination
source

202

High speed camera
Fixed focal lens
Optical filter

fistula

Vein

Artery

212

200

FIG. 2

FIG. 3

Level 0

402 — Patient places his hand inside the monitoring sleeve

404 — Automatic or semi-automatic detection of the vascular access body

406 — Recording

408 — Image processing

414 — Features extraction

410 — Database

412 — previous checks

416 — Classifier

418 — Early detection of failure

420 — Stable

FIG. 4A

Features extraction
- pulse wave velocity -

FIG. 4B

Start

Image Scaling
[pixels/mm] ◄— 422

subtraction two
consecutive
images ◄— 424

Detection
the center of two
most bright spots ◄— 426

Calculate the
geodesic distance
along the blood
vessel between
the two spots ◄— 428

divide the
distance by the
time period between
the two images ◄— 430

End

**FIG. 4C**

Features extraction
- Collateral veins -

Start

Image Scaling
[pixels/mm] ← 434

vein route
detection ← 436

Detection
branches and their
route along
the vein route ← 438

Calculate the length
of each branch
and it's geodesic
distance from fistula ← 440

calculate optional parameters:
1.Number of branches
2.Total branches length
3.Branches' Centroid
4.Centroid distance from fistula
5.Ellipse blocks ← 442

End

Features extraction
- Aneurysm and stenosis -

**FIG. 4D**

```
            ┌─────────┐
            │  Start  │
            └────┬────┘
                 │
                 ▼
        ┌──────────────────┐
        │  Image Scaling   │ ◄──── 446
        │   [pixels/mm]    │
        └────────┬─────────┘
                 │
                 ▼
        ┌──────────────────┐
        │  Vein and artery │ ◄──── 448
        │     detection    │
        └────────┬─────────┘
                 │
                 ▼
        ┌──────────────────┐
        │  Vein and artery │ ◄──── 450
        │   segmentation   │
        └────────┬─────────┘
                 │
                 ▼
   ┌──────────────────────────┐
   │  calculate the narrowest │
   │  and widest widths along │ ◄──── 452
   │ the vein and along the artery │
   └────────────┬─────────────┘
                │
                ▼
            ┌─────────┐
            │   End   │
            └─────────┘
```

EP 4 045 138 B1

# FIG. 4E

Features extraction
- Arm elevation -

Start

Image Scaling
[pixels/mm] ◄—— 456

Fistula detection ◄—— 458

Fistula segmentation ◄—— 460

Calculate the
fistula area ◄—— 462

End

FIG. 5A

FIG. 5B

**Level 1**
**-Classifier-**

602 → FEATURE: COLLATERAL VEIN

604 → FEATURE: PULSE WAVE VELOCITY

606 → FEATURE: ARM ELEVATION

608 → FEATURE: ANEURYSM AND/OR STENOSIS

610 → HISTORY FEATURES

612 → Thresholds calculation

614 → Trends calculation

616 → statistical model

618 → Decision

620 → Early failure detection

622 → Stable

FIG. 6

FIG. 7

EP 4 045 138 B1

FIG. 8A

FIG. 8B

**FIG. 9**

EP 4 045 138 B1

FIG. 10

EP 4 045 138 B1

FIG. 11

FIG. 12

FIG. 13

FIG. 14

FIG. 15A

FIG. 15B

1512

FIG. 15C

1522

| Exam Steps | Fistula (Normal) | Graft (Normal) | Flow-related Dysfunction or Poor Maturation (Abnormal) | Infection, Steal Syndrome, or Aneurysm/Pseudoaneurysm⁺ (Abnormal) |
|---|---|---|---|---|
| *Look* | Well-developed main venous outflow, no irregular/dilated areas or aneurysm formations, adequate areas of straight vein that can be used for 2-needle, rope-ladder cannulation. Vessel collapses when arm is elevated above head. | Uniform sized graft in a loop or straight configuration. No irregular areas or aneurysm or seroma formations with organized site rotation used for cannulation. | AVF with poor maturation – multiple venous outflow veins (accessory veins), poorly defined cannulation areas. *AVF:* Stenosis can occur in artery or any venous outflow vein. Look for a narrowing of the outflow vein, abnormal pulsations or aneurysm formations. *AVF or AVG:* Dilated neck veins or surface collateral veins in the arm or neck above the vascular access. | *Infection:* Redness, swelling, induration, drainage or pus. *Steal Syndrome:* Extremity / hand discoloration, skin ulceration due to poor arterial blood flow to the hand. Check nail beds, fingers and hand for unusual skin changes. *Aneurysm:* abnormal areas of dilatation with overlying skin thinning |
| *Listen* with a stethoscope | Low pitch continuous diastolic and systolic | Low pitch continuous diastolic and systolic | High pitch discontinuous systolic only | *Steal Syndrome:* AVF may have a very strong bruit |
| *Feel* with your fingers | Thrill at the arterial anastomosis and throughout the entire outflow vein that is easy to compress | Thrill strongest at the arterial anastomosis, but should be felt over entire graft and easy to compress | *AVF:* Pulse at the site of a stenotic lesion – may be water-hammer in quality and feel *AVG:* Thrill and/or pulse strong at the site of stenotic lesion pulse has a water-hammer feel. An AVG with a low intra-access blood flow feels mushy. Local area of the graft that feels mushy or irregular in shape can be a site of aneurysm formation. | *Infection:* Warm or painful to touch, swelling *Steal Syndrome:* Feel bilateral limbs (hands and fingers) and compare for the access limb to be the same as the nonaccess limb. Compare temperature, grip strength and range of motion, and any complaints of changes in sensation or pain. If the access limb has any major differences than the nonaccess limb, consider steal syndrome. |

FIG. 16A

EP 4 045 138 B1

FIG. 16B

FIG. 17

EP 4 045 138 B1

FIG. 18A

FIG. 18B

1902

1900

1904

1908

1906

FIG. 19A

1910

1914

1912

1916

FIG. 19B

EP 4 045 138 B1

2002

2004

2006

FIG. 20

2102

2104

FIG. 21A

2102

2104

FIG. 21B

2102

2104

FIG. 21C

FIG. 22A

RECEIVE A STREAM OF IMAGES
IMAGING A PATIENT'S BODY — 2222

SELECT PIXELS WITH HIGH VARIANCE
OF INTENSITY OVER DURATION
OF THE STREAM OF IMAGES — 2224

PRODUCE A VECTOR OF INTENSITY
OVER THE DURATION — 2226

TRANSFORM THE VECTOR OF INTENSITY
TO A VECTOR OF A FREQUENCY SPECTRUM — 2228

FIG. 22B

ILLUMINATE ONE OR MORE BLOOD
VESSELS THROUGH A PATIENT'S SKIN — 2302

CAPTURE AT LEAST ONE IMAGE
OF THE BLOOD VESSELS — 2304

ANALYZE THE AT LEAST ONE IMAGE — 2306

CALCULATE A PARAMETER ASSOCIATED WITH
BLOOD VESSEL FUNCTIONALITY
BASED UPON THE IMAGE ANALYSIS — 2308

FIG. 23

```
┌─────────────────────────────────────────┐
│       PRODUCE AT LEAST ONE IMAGE         │
│          OF A PATIENT ORGAN              │ ◄──── 2302
└─────────────────────────────────────────┘
                    │
                    ▼
┌─────────────────────────────────────────┐
│        ANALYZE THE AT LEAST ONE IMAGE    │ ◄──── 2304
└─────────────────────────────────────────┘
                    │
                    ▼
┌─────────────────────────────────────────┐
│        PRODUCE PARAMETER VALUES          │
│     ASSOCIATED WITH BLOOD VESSEL         │ ◄──── 2306
│            FUNCTIONALITY                 │
└─────────────────────────────────────────┘
```

FIG. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2017119258 A
- US 2014148658 A
- US 2018000354 A
- US 2015342551 A
- US 2017287132 A

### Non-patent literature cited in the description

- **BESARAB et al.** Access Monitoring Is Worthwhile and Valuable. *Blood Purif*, February 2006, vol. 24, 77-89
- **EHSAN RAJABI-JAGHARGH** ; **RUPAK K BANERJEE**. Combined functional and anatomical diagnostic endpoints for assessing arteriovenous fistula dysfunction. *World J Nephrol*, 06 February 2015, vol. 4 (1), ISSN 2220-6124, 6-18
- **LUC TURMEL-RODRIGUES**. Salvage of immature forearm fistulas for haemodialysis by interventional radiology. *Nephrol Dial Transplant.*, December 2001, vol. 16 (12), 2365-71
- **JÜRG SCHMIDLI et al.** Editor's Choice e Vascular Access: 2018 Clinical Practice Guidelines of the European Society for Vascular Surgery (ESVS). *Eur J Vasc Endovasc Surg*, 2018, vol. 55, 757e818
- **BESARAB et al.** Access Monitoring is Worthwhile and Valuable. *Blood Purification*, February 2006
- **A. KRIZHEVSKY** ; **I. SUTSKEVER** ; **G. E. HINTON**. ImageNet Classification with Deep Convolutional Neural Networks. NIPS, 2012, 1106-1114